# EUROPEAN PATENT SPECIFICATION

(11) **EP 1 932 514 B1**
(45) Date of publication and mention of the grant of the patent: **14.11.2018**
(21) Application number: 06810110.4
(22) Date of filing: 13.09.2006
(51) Int. Cl.: A61K 8/60, A61Q 5/00, A61Q 19/00, A61K 8/49, A61Q 19/08, A61Q 19/10, A61Q 19/02

(54) **PHARMACEUTICAL PREPARATION FOR EXTERNAL APPLICATION TO SKIN COMPRISING PHOSPHORYLATED SUGAR**
PHARMAZEUTISCHE ZUBEREITUNG ZUR TOPISCHEN APPLIKATION AUF HAUT MIT PHOSPHORYLIERTEM ZUCKER
PRÉPARATION PHARMACEUTIQUE DESTINÉE À UNE APPLICATION CUTANÉE EXTERNE COMPRENANT UN SUCRE PHOSPHORYLÉ

(30) Priority: 05.10.2005 JP 2005292362
(43) Date of publication of application: 18.06.2008
(73) Proprietor: Ezaki Glico Co., Ltd., Osaka-shi, Osaka 555-8502 (JP)
(72) Inventor: TANAKA, Tomoko, Higeshinada-Ku Kobe-City (JP); KAMASAKA, Hiroshi, Kawachinagano-shi Osaka 586-0038 (JP); SUGIMOTO, Kazuhisa, Sakai-shi Osaka 590-0042 (JP); NISHIMURA, Takahisa, Nara-shi Nara 631-0833 (JP); KURIKI, Takashi, Suita-shi Osaka 565-0831 (JP)
(74) Representative: Uexküll & Stolberg
(86) International application number: PCT/JP2006/318203
(87) International publication number: WO 2007/040027

(56) References cited:
- EP-A1- 0 795 319
- EP-A2- 0 719 783
- FR-A1- 2 824 733
- JP-A- 05 124 928
- JP-A- 06 279 294
- JP-A- 08 104 696
- JP-A- 10 245 316
- JP-A- 10 251 140
- JP-A- 2002 037 796
- JP-A- 2004 506 676
- JP-A- 2005 082 494
- JP-A- 2005 501 062
- JP-A- 2006 249 077
- US-A1- 2004 022 818
- US-A1- 2004 105 823

## Description

### TECHNICAL FIELD

The present invention relates to a functional external preparation according to the claims for skin containing a phosphorylated saccharide. The present invention also relates to an external preparation for skin which is excellent in skin-moisturizing effect, skin firmness and luster-improving effects, cell-activating effect, or whitening effect. The present invention also relates to an external preparation for skin that is comprehensively excellent from the points of stability as a preparation, safety or the like.

### BACKGROUND ART

The skin loses it's original water retention property and also loses contractible and flexible properties due to the influence of aging, ultraviolet irradiation, active oxygen, and the like. Decrease of skin water retention leads to wrinkles formed on the skin resulting in deterioration in appearance, and also leads to skin drying and skin roughening. In addition, improvement of skin water retention is also very important during treatment of diseases such as allergy and atopic dermatitis.

Wrinkles are caused, for example, by cell aging such as the decrease in the number of cells producing extracellular matrix of dermis and the decrease of cellular activity and by decrease and denaturation of collagen.

Conventionally, collagen, hyaluronic acid, or ascorbic acid has been applied to prevent skin wrinkle formation. Glycerol has been used when moisturizing effect is desired. However, these methods left greasy feeling upon applying and did not result in satisfactory effect. In addition, retinoic acid and α-hydroxy acids such as glycolic acid were also used as one of the treatment methods. But they need to be blended in high blending amount causing inflammation or the like. Thus, they could not be used for a long period.

Among external preparations for skin including cosmetics and quasi-drugs, such as emulsion, skin lotion, cream, shampoo, and face wash, in which a mineral is blended, there are many products containing various inorganic mineral salts, seawater, or deep sea water as a mineral source and moisturizing agent.

Accordingly, minerals are an important factor, and, for example, magnesium deficiency often results in skin itching and skin sensitization. Zinc is essential for the synthesis of proteins such as collagen in skin or in hair, and it has an action to accelerate the recovery of wound by stimulating cell metabolism. Thus, zinc has an action to alleviate inflammation such as acne or skin roughening. Zinc deficiency causes skin roughening, acne, alopecia, or the like. Calcium deficiency results in lowered skin metabolism, and leads to the loss of skin firmness. Thus, minerals are very important components for the skin.

Addition of high concentrations of inorganic mineral salts causes problems such as coloration and precipitation by reaction with other components in product. Thus, inorganic mineral salts are contained in cosmetics at low concentrations and thus, the effect by the inorganic mineral salts themselves is small and the effect to be expected is insufficiently satisfied. In addition, application of high concentrations of inorganic mineral salts occasionally resulted in skin stimulation, unpleasant feeling and problems such as skin roughening.

There are some known cosmetics that use seawater as it is, but the mineral content therein was too low to utilize the effect of minerals sufficiently. Utilization of the minerals in seawater at higher concentration was tried. However, in Japanese Laid-open Patent Publication No. 2001-48738 (Patent Document 1), seawater was dried and thus removal of sodium chloride was needed.

Various melanogenesis inhibitors have been used for whitening or prevention of darkening of the skin, as well as for prevention or improvement of spots, freckles, chloasma or the like caused by excessive exposure to ultraviolet light. Typical examples thereof include hydroquinone, arbutin, ascorbic acid and the derivatives thereof, kojic acid, and the like.

Ascorbic acid, hydroquinone and kojic acid are very weak and unstable to heat and oxidation, particularly in water, and thus, have problems such as coloration from the degradation of it in an external preparation for skin over time. Further, two derivatives thereof, magnesium ascorbyl phosphate and arbutin in which glucose is β-bonded to a phenol group on hydroquinone, are improved in their stability to heat and oxidation, but the effects thereof are still not satisfactory.
Patent Document 1: Japanese Laid-open Patent Publication No. 2001-48738 (pp. 1 to 6)
Patent Document 2: Japanese Laid-open Patent Publication No. 8-104696 (pp. 1 to 27)
Patent Document 3: Japanese Laid-open Patent Publication No. 60-78912 (pp. 1 to 5)
Patent Document 4: Japanese Laid-open Patent Publication No. 3-94692 (pp. 1 to 12)
Patent Document 5: Japanese Laid-open Patent Publication No. 3-130299 (pp. 1 to 15)
Patent Document 6: Japanese Laid-open Patent Publication No. 3-47163 (pp. 1 to 28)
Patent Document 7: Japanese Laid-open Patent Publication No. 4-134048 (pp. 1 to 9)
Patent Document 8: Japanese Patent Publication for Opposition No. 7-121853 (pp. 1 to 3) Patent document EP 795319 concerns compositions for fair skin comöprising a phosphorylated polysaccharide produced by lactic acid bacteria.

### DISCLOSURE OF THE INVENTION

### PROBLEMS TO BE SOLVED BY THE INVENTION

There was a demand for development of an effective external preparation for skin that can prevent skin aging, give practically effective wrinkling treatment method, be used safely to the skin, and supply desirable minerals in stably dissolved in the external preparation for skin safely to the skin.

As described above, many studies conducted had aimed at improving the skin-moisturizing effect and the whitening effect. However, these were not sufficient. The objective is provision of an external preparation for skin that can result in improvement of skin condition, anti-aging effect, moisturizing effect, and whitening effect, which could not attained by conventional products.

The present invention is intended to solve the aforementioned problems, and an object of the present of invention is to provide a superior external preparation for skin.

### MEANS TO SOLVE THE PROBLEMS

The present inventors conducted intensive studies under the circumstances above, and, as a result, finally found that a phosphorylated saccharide, in particular, mineral salts of a phosphorylated saccharide, had a moisturizing effect and also a remarkable action to improve skin firmness and wrinkles, dry skin and skin roughening, by increasing collagen production ability of skin fibroblast cells and that blend of a high concentration of the phosphorylated saccharide (including mineral salt) did not lead to greasy feeling, which resulted in completion of the present invention. The mineral salts of a phosphorylated saccharide have water absorption property as it is. Thus, they supply not only their moisture to skin but also prevent vaporization moisture from skin, when applied on the skin, thus preventing drying of the skin and exhibiting moisturizing effect. In addition, the phosphorylated saccharide acts on the cells to stimulate collagen production, thus having an effect of giving a moisturized skin by preventing wrinkle formation and conditioning the skin structure.

In order to solve the aforementioned problems, the present inventors conducted intensive studies, and, as a result, finally also found that combined use of the phosphorylated saccharide and a particular component (hereinafter, also described as "auxiliary component") could further increase the effects of these components. In particular, they found that it could further increase the effect of the component having a moisturizing effect or skin anti-aging effect. In addition, the moisturizing effect of the phosphorylated saccharide improves the epidermal condition and accelerates skin turn over. Since epidermal condition is improved, auxiliary components easily exert their inherent action. Further, stabilization of the auxiliary component acted by the phosphorylated saccharide allows persistence of the activity of the auxiliary component for an extended period of time and enhancement of the effect. The inventors have found that the combined use of the phosphorylated saccharide and for example a whitening agent exerted remarkable whitening effect, which resulted in completion of the present invention.

Blend of the phosphorylated saccharide to an external preparation for skin, for example, cosmetics, quasi-drugs or medicaments, such as emulsion, skin lotion, cream, cosmetic nutrient lotion, foundation, mask, shampoo, or face wash, realizes an external preparation for skin with improved moisturizing effect and collagen production-accelerating effect. Phosphorylated saccharides having multiple different mineral (mineral salt of phosphorylated saccharide) may be combined or a phosphorylated saccharide having a single kind of mineral (mineral salt of phosphorylated saccharide) may be used. In addition, combined use with other moisturizing agent or collagen production-accelerating component is more effective. Examples of the other moisturizing agents or collagen production-accelerating components include magnesium ascorbyl phosphate, sodium ascorbyl phosphate, ascorbyl sulfate esters, ascorbyl glucoside, sodium ascorbate, ascorbyl monostearate, ascorbyl palmitate, disodium ascorbyl sulfate, calcium ascorbate, glycerol, propylene glycol, 1,3-butylene glycol, dl-sodium pyrrolidone-carboxylate, sodium lactate, sorbitol, sodium hyaluronate, hyaluronic acid, urea, collagen, marine collagen, glucose, jojoba oil, amino acids, isoflavone, ceramides, chamomile extracts, sericin, polyethylene glycol, dimethyl ether, silk sericin, lithospermum root extract, Japanese angelica root extract, olive oil, trehalose, aloe extract, seaweed extract, rosemary oil, german chamomile extract, lactic acid, red algae extract, avocado extract, sponge gourd solution, scutellaria root extract, lithospermum root extract, mulberry bark extract, watercress extract, Saponaria officinalis extract, sage extract, glycine, cysteine, herb extract, squalane, royal jelly extract, lactic acid bacteria fermentation extract, rice extract, traditional Japanese and Chinese medicinal plant extracts, and the like. The combined use of the mineral salt of a phosphorylated saccharide with one or more of other moisturizing agents or collagen production-accelerating components gives synergic effect for enhancing the effects of each other. In particular, combined use of the mineral salt of a phosphorylated saccharide and ascorbic acid exert excellent synergic effect.

To achieve the objects above, the present invention provides, for example, the following means:
(Item 1)
   Use of an external preparation for improving skin firmness, wrinkles or elasticity; alleviating dry skin; delaying aging phenomena; delaying dullness or sag; moisturizing skin; or whitening skin by removing melanin and active oxygen generated by UV irradiation, said external preparation comprising an inorganic salt of a phosphorylated saccharide, wherein the saccharide moiety in the phosphorylated saccharide is a glucan or reduced glucan having the degree of polymerization of 2 or more and 100 or less.
(Item 2)
   Use according to Item 1, wherein the inorganic salt is a calcium, magnesium, potassium, zinc, iron or sodium salt.
(Item 3)
   Use according to Item 1 or 2, wherein the external preparation further comprises a moisturizing agent.
(Item 4)
   Use according to Item 3, wherein the moisturizing agent is an ascorbic acid or an ascorbic acid derivative.
(Item 5)
   Use according to any of Items 1-4, wherein the external preparation is selected from the group consisting of skin lotion, emulsion, cream, emollient cream, cold cream, vanishing cream, facial mask, gel, face pack, foundation, cake, rouge for cheek, eye shadow, lipstick, lip cream, gloss, soap, body soap, face wash, shampoo, rinse, conditioner, hair treatment, hair lotion and shaving cream.
(Item 6)
   Use according to Item 1, wherein the external preparation comprises a second component, wherein the second component is selected from the group consisting of moisturizing agents, whitening components, and antioxidants.
(Item 7)
   Use according to Item 6, wherein the second component is a moisturizing agent, and the moisturizing agent is selected from the group consisting of ascorbic acid and the derivatives thereof, vitamins other than ascorbic acid, pyridoxine derivatives, α-tocopherol derivatives, pantothenic acid derivatives, saccharides and saccharide derivatives, amino acids and the derivatives thereof, polyhydric alcohols, phenol and the derivatives thereof, collagens, hydroxycarboxylic acids and the salts thereof, hydroxysalicylic acid glycosides, hydroxysalicylic acid aliphatic ester glycosides, hydroxycinnamic acid and the derivatives thereof, caffeic acid and the derivatives thereof, crude drug extracts, natural extracts, placenta extract, oil-soluble licorice extract, ceramides, ceramide analogues, crude sugar extracts, molasses extracts, mycelia culture and the extracts thereof, urea, hinokitiol, sulfur, Azelain and the derivatives thereof, vitamin E-nicotinate and diisopropylamine dichloroacetate.
(Item 8)
   Use according to Item 6, wherein the second component is a whitening component, and the whitening component is selected from the group consisting of tyrosinase inhibitor, endothelin antagonist, α-MSH inhibitor, α-arbutin, arbutins, the salts and derivatives thereof, ascorbic acid and the derivatives thereof, ellagic acid-based compounds and the alkali-metal salts thereof, kojic acid and the derivatives thereof, resorcinol derivatives, nordihydroguaiaretic acid, teprenone, allantoin, aminoethyl compounds, alkylenediamine carboxylic acid derivatives, betaine derivatives, acylmethyltaurines, hederacoside, gymnema saponins, beet saponins, γ-pyrrone glycosides, biphenyl compounds, sodium bisulfite, fibronectins, and plant extracts.
(Item 9)
   Use according to Item 6, wherein the second component is an antioxidant, and the antioxidant is selected from the group consisting of vitamin A's, the derivatives and salts thereof; carotenoids and the derivatives thereof; vitamin B's, the derivatives and salts thereof; vitamin C's, the derivatives and salts thereof; vitamin D's, the derivatives and salts thereof; vitamin E's, the derivatives and salts thereof; trolox, the derivatives and the salts thereof; dihydroxytoluene, butylhydroxytoluene, butylhydroxyanisole, dibutyl-hydroxytoluene, α-lipoic acid, dehydro-lipoic acid, glutathione, the derivatives and salts thereof; uric acid; erythorbic acid, the derivatives and salts thereof; gallic acid, the derivatives and salts thereof; rutin, the derivatives and salts thereof; tryptophan, the derivatives and salts thereof; histidine, the derivatives and salts thereof; cysteine derivatives and salts thereof; cystine derivatives and the salts thereof; carnosine, the derivatives and salts thereof; homocarnosine, the derivatives and salts thereof; anserine, the derivatives and salts thereof; carcinin, the derivatives and salts thereof; dipeptide or tripeptide derivatives containing histidine and/or tryptophan and/or histamine and the salts thereof; flavonoids; tannic acid; caffeic acid; ferulic acid; protocatechuic acid; chalcone; oryzanol; carnosol; sesamol; sesamine; sesamolin; gingerone; curcumin; tetrahydrocurcumin; clovamide; deoxyclovamide; shogaols; capsaicin; vanillylamide; ellagic acid; bromphenol; flavoglassine; melanoidin; riboflavin; riboflavin butyrate esters; flavin mononucleotide; flavin adenine nucleotide; ubiquinone; ubiquinol; mannitol; bilirubin; cholesterol; ebselene; seleno-methionine; ceruloplasmin; transferrin; lactoferrin; albumin; bilirubin; superoxide dismutase; catalase; glutathione peroxidase; metallothionein; O-phosphono-pyridoxylidene rhodamine; N-(2-hydroxybenzyl)amino acids, the derivatives and salts thereof; and N-(4-pyridoxylmethylene)amino acids, the derivatives and salts thereof.
(Item 10)
   Use according to Item 6, wherein the phosphorylated saccharide is an inorganic salt of a phosphorylated saccharide.
(Item 11)
   Use according to Item 6, wherein the phosphorylated saccharide is a calcium, magnesium, potassium, zinc, iron or sodium salt.
(Item 12)
   Use according to Item 6, wherein the external preparation is selected from the group consisting of skin lotion, emulsion, cream, emollient cream, cold cream, vanishing cream, facial mask, gel, face pack, foundation, cake, rouge for cheek, eye shadow, lipstick, lip cream, gloss, soap, body soap, face wash, shampoo, rinse, conditioner, hair treatment, hair lotion and shaving cream.
(Item 13)
   An external preparation for use in a method of increasing skin moisturization and thus activating skin metabolism, wherein the external preparation comprises: an inorganic salt of a phosphorylated saccharide, wherein the saccharide moiety in the phosphorylated saccharide is a glucan or reduced glucan having a degree of polymerization of 2 or more and 100 or less.
(Item 14)
   An external preparation for use in a method of Item 13, wherein the external preparation comprises a second component, wherein the second component is selected from the group consisting of moisturizing components, whitening components, ultraviolet absorbents, anti-inflammatory agents, cell-activating agents and antioxidants.
(Item 15)
   An external preparation for use as a medicament comprising: an inorganic salt of a phosphorylated saccharide, wherein the saccharide moiety in the phosphorylated saccharide is a glucan or reduced glucan having the degree of polymerization of 2 or more and 100 or less.
(Item 16)
   An external preparation for use according to Item 15, wherein external preparation comprises a second component which is selected from the group consisting of moisturizing components, whitening components, ultraviolet absorbents, anti-inflammatory agents, cell-activating agents and antioxidants.

### EFFECT OF THE INVENTION

The external preparation for skin disclosed herein has an advantage of resulting in moisturized skin by giving moisturizing effect and preventing wrinkle formation by acceleration of collagen production. Furthermore, when using an inorganic salt of phosphorylated saccharide, it can stably supply the optimal kind of mineral in dissolved state at the optimal concentration to the skin.

In the external preparation for skin disclosed herein, a phosphorylated saccharide, a phosphorylated saccharide mineral salt or a substance in which a phosphorylated saccharide binds to at least one mineral selected from calcium, magnesium, potassium, zinc, iron and sodium, is contained with various auxiliary components. These phosphorylated saccharides are, even in alone, excellent in moisturizing effect as well as stability and safety as a preparation of a cell-activating agent. These various auxiliary components can synergistically enhance the inherent effects of these phosphorylated saccharides. Accordingly, the external preparation for skin disclosed herein significantly improves skin-moisturizing effect, aging prevention, cell-activating effect or whitening effect; it is excellent in stability and safety as a preparation, and thus, is very useful in cosmetic and medical applications.

### BEST MODE FOR CARRYING OUT THE INVENTION

Hereinafter, the present invention will be described in detail.

### (1. Phosphorylated saccharide)

The external preparation for skin disclosed herein contains a phosphorylated saccharide. The term "phosphorylated saccharide", as used in the present specification, means a saccharide having at least one phosphate group in the molecule . The number of the phosphate groups in the phosphorylated saccharide is not particularly limited, but preferably 10 or less, more preferably 5 or less, per phosphorylated saccharide molecule. More preferably, the number of the phosphate groups in the phosphorylated saccharide is 1, 2 or 3, particularly preferably 1 or 2, per phosphorylated saccharide molecule.

The degree of polymerization of the saccharides in the phosphorylated saccharide is preferably 2 or more, more preferably 3 or more. The degree of polymerization of the saccharides in the phosphorylated saccharide is preferably 100 or less, more preferably 90 or less, more preferably 80 or less, more preferably 70 or less, more preferably 60 or less, more preferably 50 or less, more preferably 40 or less, more preferably 30 or less, more preferably 20 or less, more preferably 10 or less, more preferably 9 or less, more preferably 8 or less, more preferably 7 or less, more preferably 6 or less, and particularly preferably 5 or less.

The molecular weight of the phosphorylated saccharide is preferably 400 or more, more preferably 500 or more, more preferably 600 or more, and particularly preferably 700 or more. The molecular weight of the phosphorylated saccharide is preferably 1,000,000 or less, more preferably 100,000 or less, more preferably 10,000 or less, particularly preferably 2,000 or less, and in one embodiment 1,000 or less.

The term "neutral saccharide", as used in the present specification, means a saccharide without any phosphate group binding. The phosphorylated saccharide may be in the free form, dissociated form, or in the salt state. Preferably, the phosphorylated saccharide is in the form of the inorganic salt. The phosphorylated saccharide is preferably an inorganic salt of a phosphorylated saccharide, and more preferably a calcium, magnesium, potassium, zinc, iron or sodium salt. The phosphorylated saccharide for use in the present invention is preferably the phosphorylated saccharide described in Japanese Laid-open Patent Publication No. 8-104696. In particular, it is possible to develop an external preparation for skin having potent collagen-producing ability and moisturizing effect, by blending a mineral salt of a phosphorylated saccharide in the external preparation for skin.

The saccharide moiety of the phosphorylated saccharide is glucan or reduced glucan. Here the reduced glucan is a glucan of which the reducing terminal aldehyde refers to reduced to its corresponding alcohol. The reduced glucan is obtained, for example, by reducing the aldehyde of glucan into alcohol by hydrogenation.

The degree of polymerization of the glucan or the reduced glucan, i.e., the number of glucose residues, is 2 or more, more preferably 3 or more. The number of glucose residues is 100 or less, preferably 90 or less, more preferably 80 or less, more preferably 70 or less, more preferably 60 or less, more preferably 50 or less, more preferably 40 or less, more preferably 30 or less, more preferably 20 or less, more preferably 10 or less, more preferably 9 or less, more preferably 8 or less, more preferably 7 or less, more preferably 6 or less, and particularly preferably 5 or less.

The term "reduced phosphorylated saccharide", as used in the present specification, means a phosphorylated saccharide having a structure of which the aldehyde on the reducing terminus is reduced to alcohol.

Since the phosphate group of the phosphorylated saccharide has a negative charge, it can bind, for example, to a positively charged atom and molecule. That is to say, the phosphorylated saccharide can bind to any positively charged inorganic ions. It should be noted that inorganic ions are also called minerals in the present specification.

In the present specification, the term "inorganic salt of phosphorylated saccharide" means a phosphorylated saccharide in the form of the inorganic salt. The inorganic salt of phosphorylated saccharide may be an alkali metal salt, an alkali-earth metal, or a transition metal salt. It is preferably an inorganic ion, which is known to have little adverse effect to the body. It may be, for example, a calcium, potassium, zinc, iron, sodium or other salt.

The number of the inorganic ions is not particularly limited. All phosphate groups present in the phosphorylated saccharide may be bound to inorganic ions, or only part of them may be bound to inorganic ions. The phosphorylated saccharide may have only one, two, three or more inorganic ions in one phosphorylated saccharide molecule. Preferably, the phosphorylated saccharide has 20 or less inorganic ions, more preferably 10 or less inorganic ions, in one phosphorylated saccharide molecule.

Phosphorylated saccharide calcium salt is known to have tooth remineralization effect, calcium absorption-accelerating effect and also taste-improving effect. However, it was not known at all that, when the phosphorylated saccharide inorganic salt is applied to an external preparation for skin such as cosmetic product, the preparation has the moisturizing effect and collagen production-accelerating effect.

In a preferred embodiment, the phosphorylated saccharide is those in which the saccharide moiety is glucan or reduced glucan, and at least one phosphoric acid is bound per one glucan molecule. In another preferred embodiment, the phosphorylated saccharide is those in which the saccharide moiety is glucan or reduced glucan, and the glucan or reduced glucan is bound to one to two phosphate groups.

In yet another preferred embodiment, the phosphorylated saccharide is those in which the saccharide moiety is glucan or reduced glucan, and the glucan is composed of 3 to 5 α-1,4-bound glucoses; and the glucan or reduced glucan is bound to one phosphate group.

In yet another preferred embodiment, the phosphorylated saccharide is those in which the saccharide moiety is glucan or reduced glucan, and the glucan or reduced glucan is composed of 2 to 8 α-1,4-bound glucoses, and the glucan or reduced glucan is bound to one or two phosphate groups.

In yet another preferred embodiment, the phosphorylated saccharide is those in which the saccharide moiety is glucan or reduced glucan, and the glucan or reduced glucan has α-1,4-bound glucoses as a main chain and α-1,6- or α-1,4-bound glucoses as a side-chain.

The phosphorylated saccharide for use in the present invention may be used as one kind of pure compound or as a mixture of multiple species. A mixture is obtained when it is prepared according to the method described in Japanese Laid-open Patent Publication No. 8-104696. The mixture may be used as it is, or only one kind of compound may be selected and used, after it is separated as a pure compound. The phosphorylated saccharide exerts its excellent performance, independently on whether it is used in one species or as a mixture.

Such a phosphorylated saccharide is a compound excellent in safety to the skin and stability as a drug over time.

The blending amount of the phosphorylated saccharide is, preferably, about 0.01 % by weight or more, more preferably, about 0.05 % by weight or more, more preferably, about 0.1 % by weight or more, with respect to the total amount of the external preparation for skin. The blending amount of the phosphorylated saccharide is preferably, about 30 % by weight or less, more preferably, about 20 % by weight or less, more preferably, about 10 % by weight or less, with respect to the total amount of the external preparation for skin.

The phosphorylated saccharide may be prepared, for example, by phosphorylation of a known saccharide. A method of producing the phosphorylated saccharide is described in Japanese Laid-open Patent Publication No. 8-104696.

The saccharides which are raw materials for the phosphorylated saccharide include glucan, mannan, dextran, agar, cyclodextrin, fucoidan, gellan gum, locust bean gum, guar gum, tamarind gum, and xanthan gum. Hereinafter, a case where glucan is used will be described. Starches in which many phosphate groups is bound, such as general crude vegetable starch, preferably crude potato starch, are preferable for production, but purified starches may also be used. Chemically modified starches can also be used preferably. Furthermore, various saccharides having chemically bound phosphate groups may also be used. In potato starch, many phosphate groups are bound via ester bonds to its constituent glucoses on positions 3 and 6. The phosphate groups are present mainly in amylopectin.

According to the invention, the saccharide is glucan, and the phosphorylated saccharide may be obtained by degradation of a phosphate group-containing starch or a phosphate group-containing chemically modified starch.

In a preferred embodiment, a starch degrading enzyme, a glycosyltransferase, an α-glucosidase, or a combination of one or more of them (excluding the use of α-glucosidase alone) is allowed to act on a phosphate group-containing starch or a phosphate group-containing chemically modified starch.

In a preferred embodiment, the starch degrading enzyme described above is a combination of one or more of α-amylase, β-amylase, glucoamylase, isoamylase, pullulanase, and neopullulanase. In a more preferable embodiment, the glycosyltransferase described above is cyclodextrin glucanotransferase.

In a preferred embodiment, in the production method described above, a glycosyltransferase is allowed to act on the phosphorylated saccharide. The glycosyltransferase described above is cyclodextrin glucanotransferase.

The derivative of the phosphorylated saccharide is produced, for example, by treating the phosphorylated saccharide or the phosphorylated saccharide derivative with a salt of alkali-earth metal or iron.

### (2. Second component)

The external preparation for skin may contain a second component (also referred to as auxiliary component in the present specification), in addition to the phosphorylated saccharide. Combined use of the phosphorylated saccharide with at least one auxiliary component allows improvement of the external preparation for skin in terms of enhancement of its skin-moisturizing effect, alleviation of dry skin and skin roughening, improvement in skin elasticity and firmness and also improvement of its whitening effect. Hereinafter the auxiliary components for use in the present invention will be described.

In addition to the following effects, these auxiliary components have the stability or an effect to improve the safety as a drug. If used in the range of blending amount described below, in combination with the phosphorylated saccharide, any auxiliary component does not affect the phosphorylated saccharide in the external preparation for skin and gives a preparation which is fine in stability over time and may exert high moisturizing and whitening effects. The blending amount may be increased or decreased according to the expected effect. It should be noted that at least one auxiliary components described below may be used, i.e., one auxiliary component may be used alone, or two or more auxiliary components may be used in combination.

The second component is preferably selected from the group consisting of moisturizing agents, whitening components, ultraviolet absorbents, anti-inflammatory agents, cell-activating agents and antioxidants.

### (2.1 Moisturizing agents)

In the present specification, the term "moisturizing agent" means a highly absorptive substance or a compound having collagen-inducing effect, that is used for moisturization and prevention of drying of skin, hair or the like.

Examples of moisturizing agents include the followings: ascorbic acid and the derivatives thereof, vitamins other than ascorbic acid, pyridoxine derivatives, α-tocopherol derivatives, pantothenic acid derivatives, saccharides and saccharide derivatives, amino acids and the derivatives thereof, polyhydric alcohols, phenol and the derivatives thereof, collagens, hydroxycarboxylic acid and the salts thereof, hydroxysalicylic acid glycosides, hydroxysalicylic acid aliphatic ester glycosides, hydroxycinnamic acid and the derivatives thereof, caffeic acid and the derivatives thereof, crude drug extracts, natural extracts, placenta extract, oil-soluble licorice extract, ceramides, ceramide analogues, crude sugar extracts, molasses extracts, mycelia culture and the extracts thereof, urea, hinokitiol, sulfur, Azelain and the derivatives thereof, vitamin E nicotinate and diisopropylamine dichloroacetate, deep sea water, and alkaline simple hot spring water.

Examples of ascorbic acid and the derivatives thereof include monoalkyl ascorbate esters such as ascorbyl monostearate, ascorbyl monopalmitate, and ascorbyl monooleate; ascorbic acid monoester derivatives such as ascorbic acid monophosphate esters and ascorbyl-2-sulfate; ascorbic acid diester derivatives such as ascorbyl distearate, ascorbyl dipalmitate, ascorbyl dioleate and ascorbic acid diphosphate esters; ascorbic acid trialkyl esters such as ascorbyl tristearate, ascorbyl tripalmitate, and ascorbyl triolate; ascorbic acid triester derivatives such as ascorbic acid triphosphate ester, ascorbic acid glycosides such as ascorbyl-2-glucoside, and the like. In particular, L-ascorbic acid, commonly called vitamin C, has cell-respiration action, enzyme-activating action, collagen-forming action by its strong reductive action and also has melanin-reducing action. The blending amount of ascorbic acid and the derivatives thereof is preferably, about 0.01 % by weight or more with respect to the total amount of the external preparation for skin, and, although there is no particular upper limit, it is preferably, for example, about 10 % by weight or less.

The vitamins other than ascorbic acid include natural extracts, retinol, retinal (vitamin A1), dehydroretinal (vitamin A2), carotene, lycopene (provitamin A), thiamine hydrochloride, thiamine sulfate (vitamin B1), riboflavin (vitamin B2), pantothenic acid (vitamin B5), pyridoxine (vitamin B6), cyanocobalamin (vitamin B12), folic acids, nicotinic acids, pantothenic acids, biotins, choline, inositols, ergocalciferol (vitamin D2), cholecalciferol (vitamin D3), dihydrotachysterol, vitamin E's such as tocopherol and the derivatives thereof, and ubiquinones, vitamin K's such as phytonadione (vitamin K1), menaquinone (vitamin K2), menadione (vitamin K3), and menadiol (vitamin K4), as well as essential fatty acids (vitamin F), carnitine, γ-oryzanol, orotic acid, vitamin P's (rutin, eriocitrin, hesperidin), and vitamin U. Desirable are retinol, retinal (vitamin A1), dehydroretinal (vitamin A2), carotene, lycopene (provitamin A), pyridoxine, ubiquinones, vitamin K's such as phytonadione (vitamin K1) and menaquinone (vitamin K2), hesperidin (vitamin P) and the derivatives thereof (such as α-glucosyl-hesperidin). These vitamins can be blended alone or in combination of two or more kinds by an appropriate selection. The blending amount of the vitamins other than ascorbic acid varies and is not determined specifically, but preferably may be about 0.001 % by weight or more, more preferably, about 10.0 % by weight or less, with respect to the total amount of the external preparation for skin.

The pyridoxine derivatives include pyridoxal, pyridoxamine, pyridoxine-5'-phosphate, pyridoxal-5'-phosphate, pyridoxamine-5'-phosphate, pyridoxal phosphate, pyridoxic acid, and the like. The blending amount of the pyridoxine derivatives is preferably, about 0.001 % by weight or more, more preferably, about 0.01 % by weight or more, with respect to the total amount of the external preparation for skin. The blending amount of the pyridoxine derivatives is preferably, about 5 % by weight or less, more preferably, about 3 % by weight or less, with respect to the total amount of the external preparation for skin.

The α-tocopherol derivatives is, for example, an ester of vitamin E acid, α-tocopheryl retinoate. In this case, the α-tocopherol is DL-α-tocopherol, D-α-tocopherol, or a natural tocopherol mixture containing D-α-tocopherol. The vitamin A acid is retinoic acid (all-trans-retinoic acid), 13-cis-retinoic acid, 11-cis-retinoic acid, 9-cis-retinoic acid, or the mixture of these isomers. In particular, the ester of DL-α-tocopherol and all-trans-retinoic acid is preferable.

The pantothenic acid derivatives include pantetheine-S-sulfonate,
4'-phosphopantetheine-S-sulfonate, pantetheine, glucopyranosyl pantothenic acid and the like. These may be used not only in the form of free acid but also in the form of salt. The salts include widely organic and inorganic acid salts. Alkali-metal and alkali-earth metal salts are preferable. The blending amount of the pantothenic acid derivatives is preferably, about 0.001 % by weight or more, more preferably, about 0.1 % by weight or more, with respect to the total amount of the external preparation for skin. The blending amount of the pantothenic acid derivatives is preferably, about 5 % by weight or less, more preferably, about 3 % by weight or less, with respect to the total amount of the external preparation for skin.

The saccharides and saccharide derivatives include maltitol, maltotriose, mannitol, sucrose, erythritol, glucose, fructose, starch hydrolysates, maltose, xylitol, hydrogenated starch hydrolysates, D-glycerylaldehyde, dihydroxyacetone, D-erythrose, D-erythrulose, D-threose, erythritol, L-arabinose, D-xylose, L-lyxose, D-arabinose, D-ribose, D-ribulose, D-xylulose, L-xylulose, D-glucose, D-talose, D-psicose, D-galactose, D-fructose, L-galactose, L-mannose, D-tagatose, aldoheptose, heplose, octuloses, 2-deoxy-D-ribose, 6-deoxy-L-galactose, 6-deoxy-L-mannoses, D-galactosamine, sialic acid, aminouronic acid, muramic acids, D-glucuronic acid, D-mannuronic acid, L-guluronic acid, D-galacturonic acid, L-iduronic acids, sucrose, guntianose, umbelliferose, lactose, planteose, isoligunoses, α,α-trehalose, raffinose, ligunoses, umbilisine, stachyose, verbascoses, mucopolysaccharides such as chitosan and chitosan degrades, hyaluronic acid, chondroitin-4-sulfate, chondroitin-6-sulfate, dermatan sulfate, mucoitin sulfate, charonine sulfate, keratosulfate, heparin, chitin and the salts thereof; and glucosamines and the derivatives thereof such as glucosamine, glucosamine-6-phosphate and glucosamine-6-sulfate. Generally, polysaccharides containing amino acids are known to have actions to smooth movement of the living organisms and protect them from external environment by covering the surface of the cell and tissue. One, or two or more kinds of these saccharide and saccharide derivative components are blended, as appropriate selected. The blending amount varies according to the kind of the saccharides and the saccharide derivative used and is not specified definitely. Typically, the blending amount of the saccharides and the saccharide derivatives is about 0.01 % by weight or more and about 5 % by weight or less, with respect to the total amount of the external preparation for skin.

Amino acids and the derivatives thereof are used to recover the hydration property of the aged or hardened epidermis. Amino acids and the derivatives thereof include neutral amino acids such as glycine, serine, cystine, alanine, threonine, cysteine, valine, phenylalanine, methionine, leucine, tyrosine, proline, isoleucine, tryptophan and hydroxyproline; acidic amino acids such as aspartic acid, asparagine, glutamine and glutamic acid; and basic amino acids such as arginine, histidine and lysine. Amino acid derivatives include acylsarcosine and the salts thereof, acylglutamic acid and the salts thereof, acyl-β-alanine and the salts thereof, glutathione, pyrrolidone carboxylic acid and the salts thereof; oligopeptides such as Glutathin, carnosine, gramcidin S, Tyrocidin A, Tyrocidin B; and γ-aminobutyric acid, γ-amino-β-hydroxybutyric acid and the salts thereof. When the blending amount of the amino acids and the derivatives thereof is too small, obtained skin-warming effect may be weak, on the other hand, when it is too large, there may be no improvement in obtained effect and it may become difficult to prevent denaturation of the amino acids. The blending amount of the amino acids and the derivatives thereof is preferably about 0.01 % by weight or more, more preferably, about 0.05 % by weight or more, with respect to the total amount of the external preparation for skin. The blending amount of the amino acids and the derivatives thereof is preferably, about 20 % by weight or less, more preferably, about 10 % by weight or less, with respect to the total amount of the external preparation for skin.

The polyhydric alcohols include dihydric polyhydric alcohols such as ethylene glycol, trimethylene glycol, tetramethylene glycol, propylene glycol, dipropylene glycol, 1,3-butylene glycol, 1,2-butylene glycol, 2,3-butylene glycol, 1,2-pentanediol, pentamethylene glycol, 2-butene-1,4-diol, hexylene glycol, and octylene glycol; trihydric polyhydric alcohols such as glycerol, trimethylolpropane, and 1,2,6-hexanetriol; quadrihydric polyhydric alcohols such as pentaerythritol; pentahydric polyhydric alcohols such as xylitol and fructose; hexahydric polyhydric alcohols such as sorbitol and mannitol; polyhydric alcohols such as hydrogenated starch hydrolysates; polyhydric alcohol polymers such as diethylene glycol, triethylene glycol, polypropylene glycol, tetraethylene glycol, diglycerol, polyethylene glycol, triglycerol, tetraglycerol, and polyglycerol; polyalkyleneoxide ethers such as POE-tetrahydrofurfuryl alcohol, POP-butyl ether, POP/POE-butyl ether, tripolyoxypropylene glycerin ether, POP-glycerin ether, POP/POE-diglycerin ether, POP-glycerin ether phosphoric acid, and POP/POE-pentaerythritol ether; and the like. The blending amount of the polyhydric alcohols is preferably, about 0.01 % by weight or more, with respect to the total amount of the external preparation for skin. There is no particular upper limit for the blending amount of the polyhydric alcohols, but it is preferably, about 10 % by weight or less.

The phenols and the derivatives thereof include hydroquinone glycoside, hydroquinone mono ethyl ether, hydroquinone mono-n-propyl ether, hydroquinone mono-n-butyl ether, hydroquinone mono-n-hexadecyl ether, hydroquinone mono-n-octadecyl ether, p-ethylphenol, p-n-propylphenol, p-n-butylphenol, p-t-butylphenol, p-isopropylphenol, p-hexadecylphenol, p-octadecylphenol, 4-isopropylcatechol mono butyl ester, 4-isopropylcatechol mono heptadeca ester, and the like. The blending amount of the phenols and the derivatives thereof is preferably, about 0.01 % by weight or more, more preferably, about 0.1 % by weight or more, with respect to the total amount of the external preparation for skin. The blending amount of the phenols and the derivatives thereof is preferably, about 20 % by weight or less, more preferably, about 10 % by weight or less, with respect to the total amount of the external preparation for skin.

The collagens include collagens extracted from mammal skin, tendon, bone, blood vessel, connective tissue, collagen fibers, or the like; fish collagens (skin or squama extracts); soluble collagens; collagen hydrolysate solution; ethyl ester of hydrolyzed collagen; hexadecyl ester of hydrolyzed collagen, and the like. The blending amount of the collagens is preferably, about 0.01 % by weight or more, with respect to the total amount of the external preparation for skin. The blending amount of the collagens has no particular upper limit, but it is preferably 1.0 % by weight or less.

The hydroxycarboxylic acids and the salts thereof include glycolic acid, lactic acid, malic acid, tartaric acid, citric acid, salicylic acid, mevalonic acid, mevalonic acid lactone, and the like, and the salts thereof include metal salts such as of sodium, potassium, and magnesium; organic salts such as triethanolamine and 2-amino-methyl-1, 3-propanediol, and the like. The blending amount of the hydroxycarboxylic acids and the salts thereof is preferably, about 0.0001 % by weight or more, more preferably, about 0.001 % by weight or more, with respect to the total amount of the external preparation for skin. The blending amount of the hydroxycarboxylic acids and the salts thereof is preferably about 5 % by weight or less, more preferably, about 3 % by weight or less, with respect to the total amount of the external preparation for skin about.

The hydroxysalicylic acid glycosides and the glycosides of hydroxysalicylic acid aliphatic esters are represented by General Formulae 1, 2, and 3. These glycosides can be obtained by reacting a hydroxysalicylic acid or a hydroxysalicylic acid aliphatic ester with an acetylated saccharide such as pentaacetylglucose (or acetobromo sugars such as acetobromoglucose) in the presence of an acid catalyst. The blending amount of the hydroxysalicylic acid glycosides or the glycosides of hydroxysalicylic acid aliphatic esters is preferably, about 0.001 % by weight or more, more preferably, about 0.1 % by weight or more, with respect to the total amount of the external preparation for skin. The blending amount of the hydroxysalicylic acid glycosides or the glycosides of hydroxysalicylic acid aliphatic esters is preferably, about 20 % by weight or less, more preferably, about 7 % by weight or less, with respect to the total amount of the external preparation for skin.

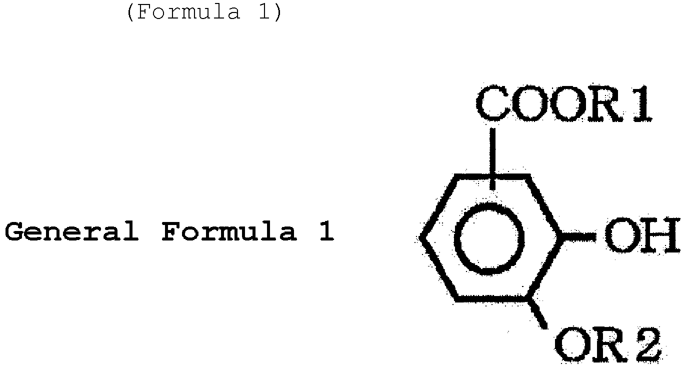

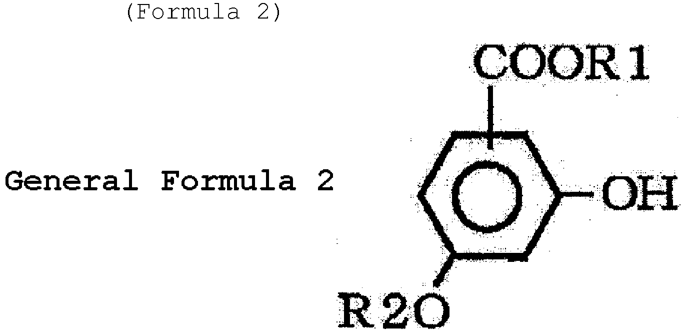

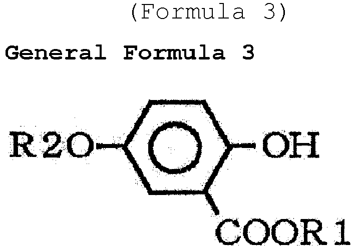

In Formulae 1 to 3, R1 is a hydrogen atom or a saturated or unsaturated, straight or branched hydrocarbon group having 1 to 20 carbons; and R2 is a saccharide residue.

Specific examples of the glycosides described above include 3-β-D-glucopyranosyloxysalicylic acid, methyl 3-β-D-glucopyranosyloxysalicylate, ethyl 3-β-D-glucopyranosyloxysalicylate, propyl 3-β-D-glucopyranosyloxysalicylate, isopropyl 3-β-D-glucopyranosyloxysalicylate, 4-β-D-glucopyranosyloxysalicylic acid, methyl 4-β-D-glucopyranosyloxysalicylate, ethyl 4-β-D-glucopyranosyloxysalicylate, propyl 4-β-D-glucopyranosyloxysalicylate, isopropyl 4-β-D-glucopyranosyloxysalicylate, 5-β-D-glucopyranosyloxysalicylic acid, methyl 5-β-D-glucopyranosyloxysalicylate, ethyl 5-β-D-glucopyranosyloxysalicylate, propyl 5-β-D-glucopyranosyloxysalicylate, isopropyl 5-β-D-glucopyranosyloxysalicylate, 6-β-D-glucopyranosyloxysalicylic acid, methyl 6-β-D-glucopyranosyloxysalicylate, ethyl 6-β-D-glucopyranosyloxysalicylate, propyl 6-β-D-glucopyranosyloxysalicylate, isopropyl 6-β-D-glucopyranosyloxysalicylate, 2-β-D-glucopyranosyloxy-3-hydroxybenzoic acid, methyl 2-β-D-glucopyranosyloxy-3-hydroxybenzoate, ethyl 2-β-D-glucopyranosyloxy-3-hydroxybenzoate, propyl 2-β-D-glucopyranosyloxy-3-hydroxybenzoate, isopropyl 2-β-D-glucopyranosyloxy-3-hydroxybenzoate, 2-β-D-glucopyranosyloxy-4-hydroxybenzoic acid, methyl 2-β-D-glucopyranosyloxy-4-hydroxybenzoate, ethyl 2-β-D-glucopyranosyloxy-4-hydroxybenzoate, propyl 2-β-D-glucopyranosyloxy-4-hydroxybenzoate, isopropyl 2-β-D-glucopyranosyloxy-4-hydroxybenzoate, 2-β-D-glucopyranosyloxy-5-hydroxybenzoic acid, methyl 2-β-D-glucopyranosyloxy-5-hydroxybenzoate, ethyl 2-β-D-glucopyranosyloxy-5-hydroxybenzoate, propyl 2-β-D-glucopyranosyloxy-5-hydroxybenzoate, isopropyl 2-β-D-glucopyranosyloxy-5-hydroxybenzoate and the like.

These ether compounds can be produced by a known method, for example, by direct etherification method of a corresponding alcohol with an alkylhalide, addition reaction of a corresponding alcohol and an olefin in the presence of a Lewis acid catalyst, a reduction method of an allyl ether obtained in addition reaction of a corresponding alcohol and an alkyl halide in the presence of an alkali catalyst, reduction method of an acetal or ketal produced from a corresponding alcohol and an aldehyde or a ketone, or the like. The blending amount of the ether compounds is preferably about 0.01 weight or more, more preferably, about 0.01 % by weight or more, more preferably, about 0.1 % by weight or more, with respect to the total amount of the external preparation for skin. The blending amount of the ether compounds is preferably, about 50 % by weight or less, more preferably, about 20 % by weight or less, more preferably, about 10 % by weight or less, with respect to the total amount of the external preparation for skin.

The hydroxycinnamic acid and the derivatives thereof include p-coumalic acid, hydroxycinnamic acid including p-coumaric acid and caffeic acid, and the like. The blending amount of the hydroxycinnamic acid and the derivatives thereof is preferably about 0.001 % by weight or more, more preferably, about 0.1 % by weight or more, with respect to the total amount of the external preparation for skin. The blending amount of the hydroxycinnamic acid and the derivatives thereof is preferably about 5 % by weight or less, more preferably, about 3 % by weight or less, with respect to the total amount of the external preparation for skin.

The blending amount of caffeic acid and the derivatives thereof is preferably, about 0.001 % by weight or more, more preferably, about 0.1 % by weight or more, with respect to the total amount of the external preparation for skin. The blending amount of caffeic acid and the derivatives thereof is preferably about 5 % by weight or less, more preferably, about 3 % by weight or less, with respect to the total amount of the external preparation for skin.

The crude drug extracts include mulberry (mulberry bark), peony root, scutellaria root, german chamomile, Japanese angelica root, rosemary, geranium herb, lithospermumroot, teaplant, puerariaroot, clove, licorice, loquat, better orange peel, ginseng, peony root, crataegus, ophiopogen tuber, ginger, pinecone, magnolia bark, gambir, scutellaria root, aloe, marshmallow, Spiraea japonica, watercress, cinchona bark, comfrey, scopolia, jojoba, swertia herb, yarrow (Achillea millefolium Linn'e (Compositae)), and the like, and the extracts thereof may be used similarly. In the present invention, the crude drugs and the extracts thereof mean fine powders, dried as needed, of the entire plant, root, leaf, flower, or seed of the above described crude drugs; extract solution obtained by immersion and extraction with water and/or an organic solvent and filtering off the residue; substances obtained from the extract liquid by removing solvent or the fine powders thereof; and those obtained from the above described extract liquid or the solvent removed substance by dissolving, dispersing, or diluting with an appropriate solvent. The blending amount of the crude drug extracts is preferably, about 0.0001 % by weight or more, more preferably, about 0.01 % by weight or more, with respect to the total amount of the external preparation for skin. The blending amount of crude drug extracts is preferably, about 20 % by weight or less, more preferably, about 10 % by weight or less, with respect to the total amount of the external preparation for skin.

Natural extracts include natural extracts obtained by extraction or hydrolysis of plants and animals, microorganisms and a part thereof with an organic solvent, an alcohol, a polyhydric alcohol, water, or an aqueous alcohol. The plants and animals, microorganisms and a part thereof include yeasts such as Saccharomyces; bacteria; human umbilical cord, yeast, milk-derived protein, silk, wheat, soy bean, bovine blood, swine blood, cockscomb, almond, cacao, macadamia nuts, olive, ginger, corn, linden, pine, peppermint, burdock, sesame, prune, Houttuynia cordata, Sasa veitchii, camellia, grapefruit, mallow, rice, avocado, cactus, lavender, sunflower, Japanese cypress, sesame, lily, Citrus junos, rose, acerola, cucumber, rice, shea butter, white birch, tomato, garlic, witch-hazel, sponge gourd, hop, peach, apricot, lemon, kiwifruit, Houttuynia cordata, capsicum, Sophora flavescens, Rumex japonicus, Nuphar japonicum, sage, yarrow, mallow, cnidium rhizome, swertia herb, thyme, birch, common Horsetail, sponge gourd, marronnier, Saxifraga stolonifera, arnica, lily, Artemisia princeps, phellodendron bark, safflower, gardenia fruits, jujube, citrus unshiu peel, coix seed, gardenia jasminoides, sawara cypress, chamomile, melissa, loquat, jatoba, and the like. The blending amount of the natural extracts is preferably, about 0.001 % by weight or more, preferably, about 10 % by weight or less, with respect to the total amount of the external preparation for skin.

Examples of the placenta extracts include extract liquids obtained by immersing and extracting an animal placenta such as of human, monkey, cow, pig, sheep, or mouse with water and/or an organic solvent and filtering off the residues; substances obtained from the extract liquid by removing solvent or the fine powders thereof; those obtained from the above described extract liquid or the solvent removed substance by dissolving, dispersing, or diluting with an appropriate solvent. Specifically, these are commercially available as water-soluble and oil-soluble placenta extracts. The blending amount of the placenta extract is preferably, about 0.001 % by weight or more, more preferably, about 0.1 % by weight or more, with respect to the total amount of the external preparation for skin. The blending amount of the placenta extract is preferably, about 5 % by weight or less, more preferably, about 3 % by weight or less, with respect to the total amount of the external preparation for skin.

The oil-soluble licorice extract includes an extract of licorice (scientific name: Glycyrrhiza glabra Linne), a leguminous perennial plant, extracted with extraction solvent, for example, a lower monohydric alcohol such as methyl alcohol and ethyl alcohol or a liquid polyhydric alcohol such as glycerol, propylene glycol or 1,3-butylene glycol. The preparation method thereof is not particularly limited. For example, it is extracted with various appropriate solvents at low temperature, room temperature or elevated temperature. An example of the preferred extraction method is a method by extracting with ethyl alcohol under heating for 2 to 10 hours, filtering the extract, leaving the filtrate for 2 to 3 more days such that the filtrate is aged, and then again filtrating it. If needed, the filtrate may be extracted under heating, and then concentrated to drying. The oil-soluble licorice extract thus obtained is dark brown substance having a characteristic odor. It can be utilized as it is in many cases, but, if needed, the extract may be used after purification treatment, for example, deodorization and decolorizing, in the range that does not impair the efficacy of the extract. For example, as the means for purification treatment, an activated carbon column may be used. Any purification means that are applied usually for extracted substances may be selected and used. The blending amount depends on, for example, the quality of the extract used. In a particular case, the blending amount of the oil-soluble licorice extract is preferably, about 0.0001 % by weight or more, more preferably, about 0.001 % by weight or more, with respect to the total amount of the external preparation for skin. In a particular case, the blending amount of the oil-soluble licorice extract is preferably, about 5 % by weight or less, more preferably, about 3 % by weight or less, with respect to the total amount of the external preparation for skin.

Ceramides and ceramide analogues have moisturizing, softening, whitening, anti-inflammatory, anti-oxidative, blood flow-accelerating and the like to the skin. The ceramides are represented by General Formula 4. The ceramide analogues are represented by General Formulae 5, 6, 7, 8, and 9. One or more kind of the ceramides and one or more kind of the ceramide analogues may be used alone or in combination of two or more kinds. The blending amount of the ceramides and the ceramide analogues is preferably, about 0.01 % by weight or more, more preferably, about 0.05 % by weight or more, more preferably, about 0.1 % by weight or more, with respect to the total amount of the external preparation for skin. The blending amount of the ceramides and the ceramide analogues is preferably, about 50 % by weight or less, more preferably, about 20 % by weight or less, more preferably, about 10 % by weight, with respect to the total amount of the external preparation for skin. When the blending amount is in the range above, the external preparation for skin has a particularly excellent impression, moisturizing effect, prevention and improvement of skin roughening, and stability.

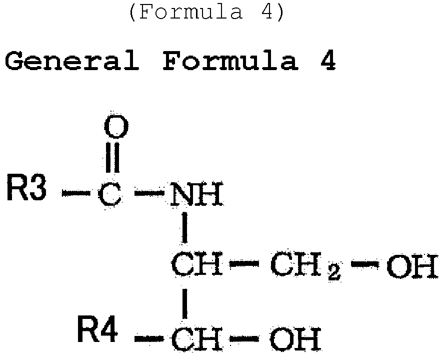

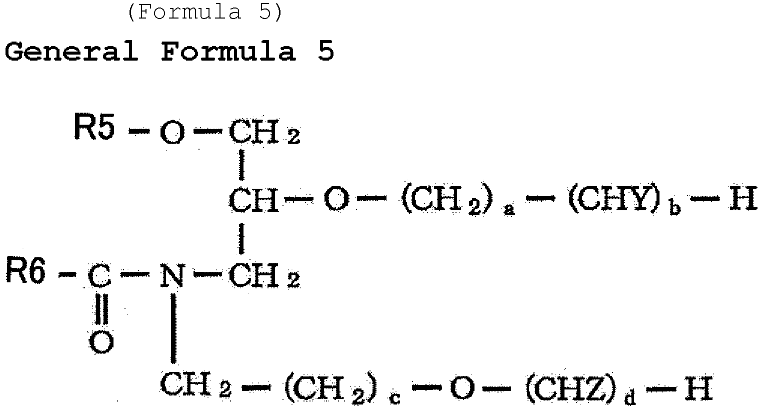

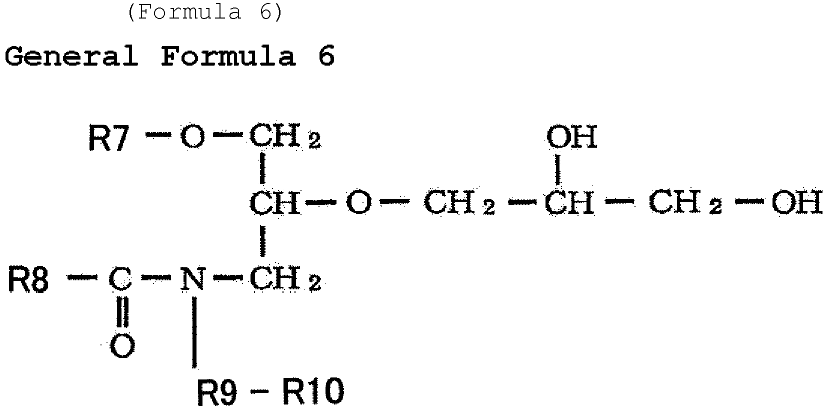

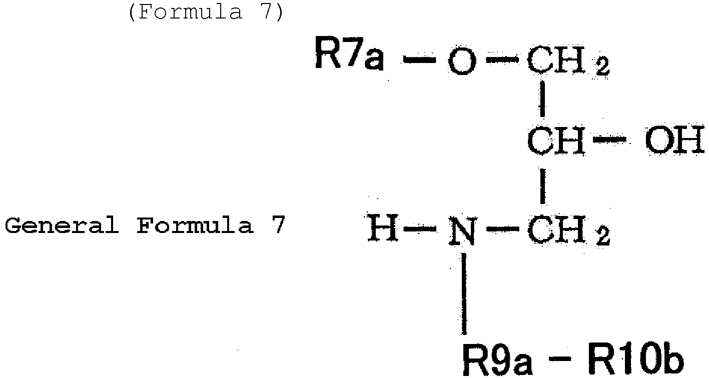

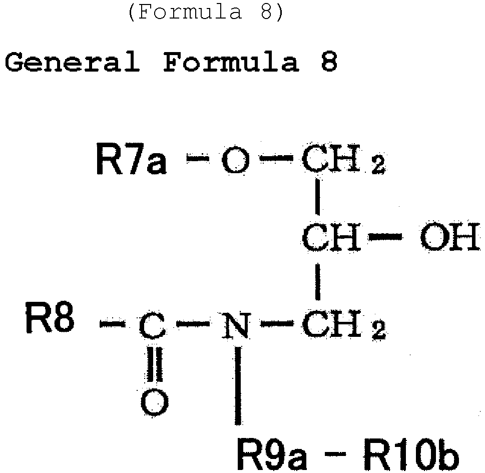

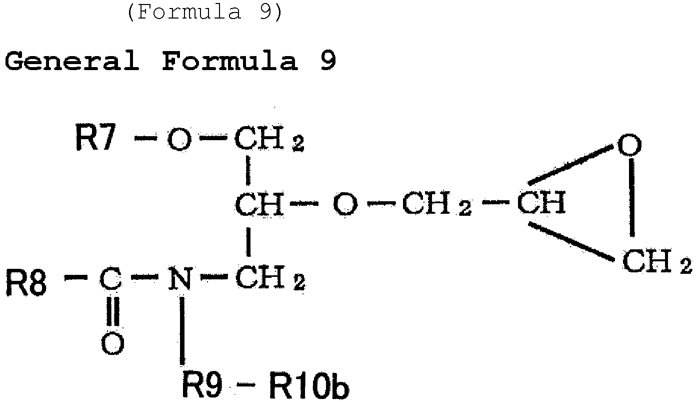

In General Formula 4, R3 and R4 are same or different, and are a hydroxyl-substituted or unsubstituted straight or branched, saturated or unsaturated hydrocarbon group having 8 to 26 carbons. In General Formula 5, R5 is a straight or branched, saturated or unsaturated hydrocarbon group having 10 to 26 carbons; R6 is a straight or branched, saturated or unsaturated hydrocarbon group having 9 to 25 carbons; each of Y and Z is a hydrogen atom or a hydroxyl group; **a** is 0 or 1; **c** is an integer of 0 to 4; and each of **b** and **d** is an integer of 0 to 3. In General Formula 6, R7 and R8 may be same or different, and are a straight or branched, saturated or unsaturated hydrocarbon group having 1 to 40 carbons, and the hydrocarbon group may be hydroxylated; R9 is a straight or branched alkylene group having 1 to 6 carbons, or a single bond; R10 is a hydrogen atom, a straight or branched alkoxy group having 1 to 12 carbons, or a 2,3-dihydroxypropyloxy group; and R10 is a hydrogen atom when R9 is a single bond. In General Formula 7, R7a is a hydrocarbon group having 4 to 40 carbons, wherein the hydrocarbon group may be hydroxylated; R9a is a straight or branched alkylene group having 3 to 6 carbons; and R10b is a straight or branched alkoxy group having 1 to 12 carbons . In General Formula 8, R7, R8, R8a, and R9a are the same as those described above. In General Formula 9, R7, R8, and R9 are the same as those above; R10b is a hydrogen atom, a straight or branched alkoxy group having 1 to 12 carbons or a 2,3-dihydroxypropyloxy group; and R10b is a hydrogen atom when R9 is a single bond.

The ether compounds represented by General Formula R11-O-(X-O) n-R12 have an action to enhance the percutaneous absorption property of the external preparation for skin and moreover is not irritant to the skin. In the General Formula, R11 and R12 maybe same or different, and is a straight, branched or cyclic alkyl group having 1 to 12 carbons, preferably 2 to 22, more preferably 3 to 20; at least one of R11 and R12 is preferably branched at two or more, particularly two sites, and particularly include methyl, butyl, n-butyl, n-decyl, n-dodecyl, n-tetradecyl, n-octadecyl, n-eicosyl, n-tetracosyl, 1-methylpropyl, 3-methylhexyl, 2-methylheptadecyl, 1,3-dimethylbutyl, 1,3-dimethylpentyl, cyclopentyl group and the like. X is an alkylene group having 1 to 12 carbons, preferably 1 to 8 carbons, and specifically includes methylene, ethylene, butylene groups and the like. The total carbon number of R11, R12 and X is essentially to be 10 to 32, preferably 12 to 28. n is 0 or 1, preferably 0.

The crude sugar extract is a brownish colorant component. The dry powder thereof is hygroscopic and has a slightly burning smell and a slightly bitter taste. The production method is described in Japanese Laid-open Patent Publication No. 60-78912. Specifically, it is produced by dissolving a crude sugar (raw sugar) or a molasses (byproduct generated during production process of sugar from raw sugar) in an appropriate amount of water, bringing it into contact with adsorbent such as non-polar polystyrene-based resin adsorbent to allow the adsorption of the colorant components, washing the adsorbent to remove the sugar components thoroughly, eluting the colorant components adsorbed on the adsorbent with an aqueous alcohol at a concentration of 20% or more, and purifying the eluted materials by concentration, freeze drying or vaporization to dryness and also recrystallization as needed. The blending amount of the crude sugar extract is preferably about 0.01 % by weight or more, more preferably, about 0.1 % by weight or more, with respect to the total amount of the external preparation for skin. The blending amount of the crude sugar extract is preferably about 10 % by weight or less, more preferably, about 5 % by weight or less, with respect to the total amount of the external preparation for skin.

The principal components of molasses extract is oligosaccharides. The molasses extract is obtained by subjecting a molasses to cold or hot immersion with a lower alcohol such as methanol or ethanol, and followed by filtration, concentration and decolorization of it. The blending amount of the molasses extract is preferably, about 0.01 % by weight or less, more preferably, about 0.1 % by weight or more, with respect to the total amount of the external preparation for skin. The blending amount of the molasses extract is preferably, about 10 % by weight or more, more preferably, about 5 % by weight or more, with respect to the total amount of the external preparation for skin.

The mycelia culture or the extract thereof is a culture of a mycelium such as mushroom or Ganodermataceae in an appropriate medium. It may be a culture liquid as it is in the case of liquid culture, or fine powder obtained by pulverizing the obtained mycelium which is dried as necessary in the case of solid culture. The mycelia culture extract means an extract obtained by subjecting the above described mycelium culture liquid, the mycelium or the fine powder thereof to immersion extraction with water and/or an organic solvent and filtering off the residue; substances obtained from the extract solution by removing solvent or the fine powders thereof; or those obtained from the above described extract solution or the solvent removed substance by dissolving, dispersing, or diluting with a appropriate solvent. The blending amount of the mycelia culture and its extract is preferably, about 0.001 % by weight or more, more preferably, about 0.1 % by weight or more, with respect to the total amount of the external preparation for skin. The blending amount of the mycelia culture and its extract is preferably, about 5 % by weight or less, more preferably, about 3 % by weight or less, with respect to the total amount of the external preparation for skin.

The blending amount of urea is preferably, about 0.1 % by weight or more, more preferably, about 1.0 % by weight or more, with respect to the total amount of the external preparation for skin. The blending amount of urea is preferably, about 20 % by weight or less, more preferably, about 10% weight or less, with respect to the total amount of the external preparation for skin.

The blending amount of hinokitiol is preferably, about 0.001 % by weight or more, more preferably, about 0.1 % by weight or more, with respect to the total amount of the external preparation for skin. The blending amount of hinokitiol is preferably, about 5 % by weight or less, more preferably, about 3 % by weight or less, with respect to the total amount of the external preparation for skin.

The blending amount of sulfur is about 0.001 % by weight or more, more preferably, about 0.1 % by weight or more, with respect to the total amount of the external preparation for skin. The blending amount of sulfur is about 5 % by weight or less, more preferably, about 3 % by weight or less, with respect to the total amount of the external preparation for skin.

The Azelain and the derivatives thereof include Azelain, azelaic acid and the like. The blending amount of the Azelain and the derivatives thereof is preferably, about 0.001 % by weight or more, more preferably, about 0.1 % by weight or more, with respect to the total amount of the external preparation for skin. The blending amount of the Azelain and the derivatives thereof is preferably, about 5 % by weight or less, more preferably, about 3 % by weight or less, with respect to the total amount of the external preparation for skin.

Vitamin E-nicotinate and diisopropylamine dichloroacetate have blood flow-accelerating action and cell-activating action, accelerating skin metabolism and preventing skin aging by damage from ultraviolet. The blending amount of vitamin E-nicotinate is preferably, about 0.01 % by weight or more, preferably, about 5 % by weight or less with respect to the total amount of the external preparation for skin. The blending amount of diisopropylamine dichloroacetate is preferably, about 0.01% by weight or more, preferably, about 5 % by weight or less with respect to the total amount of the external preparation for skin.

The deep sea water is seawater obtained from the region 200 m or more below the sea level of ocean. The deep sea water is characterized in that temperature of it is stably low, it has eutrophic property and it is clean. The blending amount of the deep sea water is preferably, about 0.05 % by weight or more, more preferably, about 0.5 % by weight or more, with respect to the total amount of the external preparation for skin. The blending amount of the deep sea water is preferably, about 30 % by weight or less, more preferably, about 10 % by weight or less, with respect to the total amount of the external preparation for skin.

The alkaline simple hot spring water means hot spring water at a temperature of 25°C or higher wherein the contents of solid components and free carbonic acid is less than 1,000 mg per 1 kg of water and has a pH of 8.5 or more and less than 10. The blending amount of the alkaline simple hot spring water is preferably, about 0.05 % by weight or more, more preferably, about 0.5 % by weight or more, with respect to the total amount of the external preparation for skin. The blending amount of the alkaline simple hot spring water is preferably, about 30 % by weight or less, more preferably, about 10 % by weight or less, with respect to the total amount of the external preparation for skin.

### (2.2 Whitening component)

The term "whitening component", as used in the present specification, means a component capable to safely induce decreasing of melanin dye when used to the skin area with increased melanin dye.

Examples of the whitening agents include the followings: tyrosinase inhibitor, endothelin antagonist, α-MSH inhibitor, α-arbutin, arbutin and the salts and derivatives thereof, ascorbic acid and the derivatives thereof, ellagic acid-based compounds and the alkali-metal salts thereof, kojic acid and the derivatives thereof, resorcinol derivatives, nordihydroguaiaretic acid, teprenone, allantoin, aminoethyl compounds, alkylenediaminecarboxylic acid derivatives, betaine derivatives, acylmethyltaurine, hederacoside, gymnema saponin, beet saponin, γ-pyrrone glycoside, biphenyl compounds, sodium bisulfite, fibronectin, plant extracts, and phenol and the derivatives thereof.

A transparent solution derived from the homogenate of a culture or the dried substance thereof is used as a tyrosinase inhibitor. The culture is obtained by inducing callus from cultured cells of Catharanthus roseus L. (cells or organ peaces, such as seedling (plantlet) root, hypocotyl, cotyledon, mature root, stem, petiole, flower, and pollen, of Catharanthus roseus L.) with a medium containing added plant growth-regulating substances including phytohormones such as auxin and cytokinin, or forming a tumor tissue from the cultured cells of Catharanthus roseus L. using Agrobacterium tumefaciens, Agrobacterium rhizogenes, or the like, and culturing the callus or the tumor tissue in a culture medium (such as Murashige and Skoog medium, Linsmaier and Skoog medium, White medium, Gamborg medium, Nitsch medium, Heller medium, Schenk and Hildebrand medium, Nitsch-Nitsch medium, or Kohlenbach and Schmidt medium) which contains hydroquinone-α-D-glucose. The blending amount may be an amount showing a desired anti-tyrosinase activity.

The endothelin antagonists are BE-18257 and the like (Japanese Laid-open Patent Publication No. 3-94692), WS7338 and the like (Japanese Laid-open Patent Publication No. 3-130299), anthraquinone derivatives (Japanese Laid-open Patent Publication No. 3-47163), and TAN-1415 and the like (Japanese Laid-open Patent Publication No. 4-134048). The blending amount of the endothelin antagonist is preferably, about 0.001 % by weight, more preferably, about 0.05 % by weight or more, with respect to the total amount of the external preparation for skin. The blending amount of the endothelin antagonist is preferably, about 10 % by weight or less, more preferably, about 5 % by weight or less, with respect to the total amount of the external preparation for skin.

The α-MSH inhibitor means a group of substances inhibiting production of α-melanotropin that are involved deeply in melanin synthesis . The blending amount of the α-MSH inhibitor is preferably, about 0.001 % by weight or more, more preferably, about 0.05 % by weight or more, with respect to the total amount of the external preparation for skin. The blending amount of the α-MSH inhibitor is preferably, about 30 % by weight or less, more preferably, about 10 % by weight or less, with respect to the total amount of the external preparation for skin.

Examples of the α-arbutin, arbutin, the salts and derivatives thereof include α-arbutin, arbutin, α-arbutin-α-glucoside, and α-arbutin-α-maltoside. The blending amount of α-arbutin, arbutin, the salts and derivatives thereof is preferably, about 0.001 % by weight or more, more preferably, about 0.05 % by weight or more, with respect to the total amount of the external preparation for skin. The blending amount of α-arbutin, arbutin, the salts and derivatives thereof is preferably, about 10 % by weight or less, more preferably, about 3 % by weight or less, with respect to the total amount of the external preparation for skin.

Examples of ascorbic acid and the derivatives thereof to be used as whitening agents include monoalkyl ascorbate esters such as ascorbyl monostearate, ascorbyl monopalmitate, and ascorbyl monooleate; ascorbic acid monoester derivatives such as ascorbic acid monophosphate esters and ascorbyl-2-sulfate; ascorbic acid diester derivatives such as ascorbyl distearate, ascorbyl dipalmitate, ascorbyl dioleate and ascorbic acid diphosphate esters; ascorbic acid trialkyl esters such as ascorbyl tristearate, ascorbyl tripalmitate, and ascorbyl trioleate; ascorbic acid triester derivatives such as ascorbic acid triphosphate ester, ascorbic acid glycosides such as ascorbyl-2-glucoside, and the like. In particular, L-ascorbic acid, commonly called vitamin C, has cell-respiration action, enzyme-activating action, collagen-forming action by its strong reductive action, and also melanin-reducing action. The blending amount of ascorbic acid and the derivatives thereof is preferably, about 0.01 % by weight or more, with respect to the total amount of the external preparation for skin. The blending amount of the ascorbic acid and the derivatives thereof has no particular upper limit, but preferably about 10 % by weight or less.

Ellagic acid-based compound and the alkali-metal salts thereof are those for improvement in long-term storage stability of the external preparation for skin according to the present invention, and represented by General Formula 10. The alkali-metal salts of the ellagic acid-based compound includes a Na and K salts. The blending amount of ellagic acid-based compound and the alkali-metal salts thereof is preferably, about 0.001 % by weight or more, more preferably, about 0.05 % by weight or more, with respect to the total amount of the external preparation for skin. The blending amount of the ellagic acid-based compound and the alkali-metal salts thereof is preferably, about 30 % by weight or less, more preferably, about 10 % by weight or less, with respect to the total amount of the external preparation for skin.

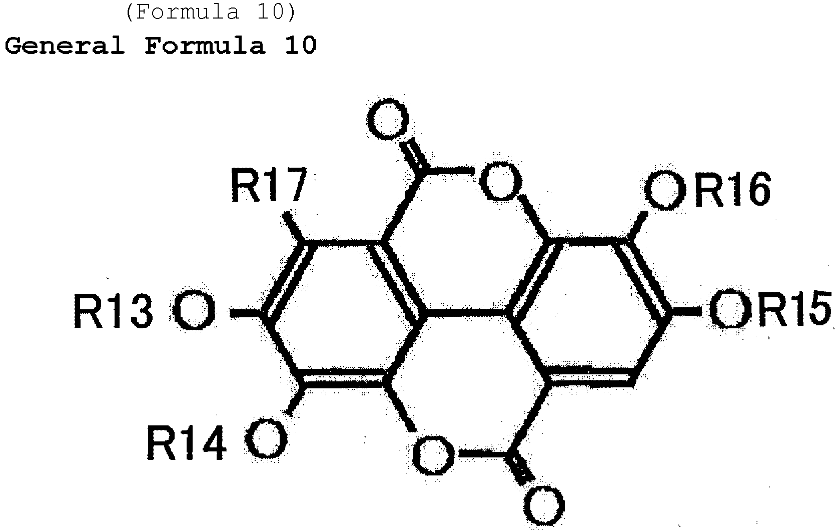

In Formula 10, R13, R14, R15, and R16 are a hydrogen atom, an alkyl group having 1 to 20 carbons (e.g., methyl group, ethyl group, propyl group, and the like), an acyl group having 1 to 20 carbon atoms (e.g., acetyl group, propionyl group, etc.), a polyoxyalkylene group represented by -(CₘH₂ₘ-O)ₙH (m is 2 or 3, n is an integer of 1 or more, particularly preferably 5 to 40) (e.g., polyoxyethylene group and polyoxypropylene group), or the saccharide residue represented by General Formula 11 and they may be the same or different one another. R17 is a hydrogen atom, a hydroxyl group, or an alkoxy group having 1 to 8 carbons.

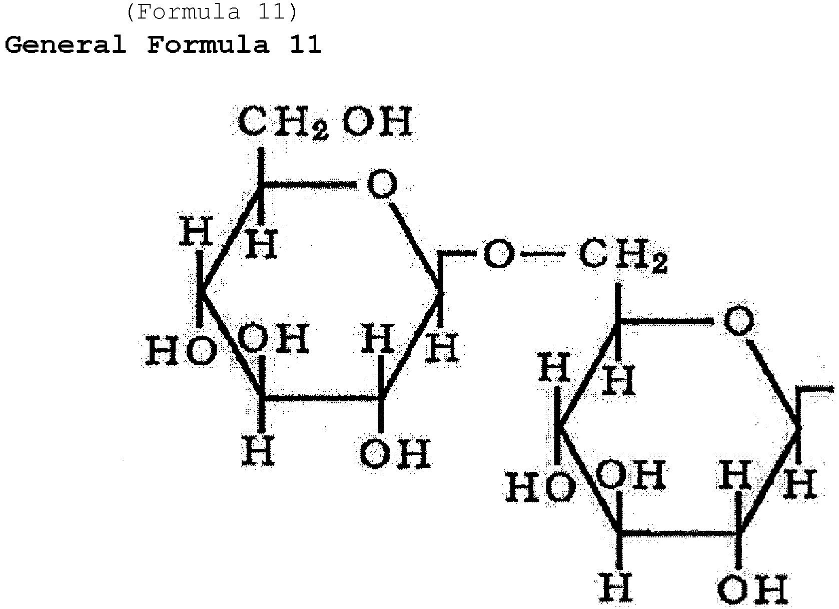

The ellagic acid-based compounds and the alkali metal salts thereof include ellagic acid, 3,4-di-o-methylellagic acid, 3,3'-di-o-methylellagic acid, 3,3',4-tri-o-methylellagic acid, 3,3',4,4'-tetra-o-methyl-5-methoxyellagic acid, 3-o-ethyl-4-o-methyl-5-hydroxyellagic acid, amritoside, and the alkali-metal salts thereof. These ellagic acid-based compounds may be obtained from natural products such as strawberry, CaesalupiniaSpinosa, eucalyptus, apple, Coriaria japonica, radiata pine, bearberry, pomegranate, Phyllanthus emblica, Sapium sebiferum leaf, Rhus javanica leaf, Uncaria gambir Acacia catechu, Platycarya strobilacea leaf, Medicine Terminalia Fruit Extract, Camptotheca acuminate, Polygonum bistorta, Lagerstroemia subcostata, Sapium discolor root, Sapium discolor leaf, Bischofia javanica, Weiderich Lythrum salicaria, Geranium pratense, Euphorbia hirta, Eucalyptus citriodora leaf, Euphorbia royleana, Psidium guajava immature fruit, Psidium guajava cortex, Mangifera indica L., nutgalls, Syzygium cumini fruit, Syzygium cumini cortex, Phyllanthus emblica root, Phyllanthus emblica cortex, Phyllanthus emblica leaf, Agrimoniae herba root, Psidium guajava leaf, Sapium sebiferum root cortex, SHIDOKON (kanppo name), CHINSYUSO (kanppo name), and geranium herb.

The kojic acid and the derivatives thereof include kojic acid; kojic acid glycosides; monoesters such as kojic acid monobutyrate, kojic acid monocaprate, kojic acid monopalmitate, kojic acid monostearate, kojic acid monocinnamate, and kojic acid monobenzoate; diesters such as kojic acid dibutyrate, kojic acid dipalmitate, kojic acid distearate, and kojic acid dioleate; and the like. The blending amount of kojic acid and the derivatives thereof is preferably, about 0.001 % by weight or more, more preferably, about 0.05 % by weight or more, more preferably, about 0.01 % by weight or more, with respect to the total amount of the external preparation for skin. The blending amount of kojic acid and the derivatives thereof is preferably, about 30 % by weight or less, more preferably, about 10 % by weight or less, more preferably, about 5 % by weight or less, with respect to the total amount of the external preparation for skin.

The resorcinol derivatives have blood flow-accelerating action and cell-activating action, preventing drying of epidermis , accelerating skin metabolism and preventing aging of epidermis by damage from ultraviolet. Specific examples thereof include 4-n-ethylresorcinol, 4-n-butylresorcinol, 4-n-hexylresorcinol, 4-isoamylresorcinol and the like. The blending amount of the resorcinol derivatives is preferably, about 0.0001 % by weight or more, more preferably, about 0.01 % by weight or more, with respect to the total amount of the external preparation for skin. The blending amount of the resorcinol derivatives is preferably, about 20 % by weight or less, preferably, about 10 % by weight or less, with respect to the total amount of the external preparation for skin.

Nordihydroguaiaretic acid is a substance generally known as an antioxidant or a lipoxygenase inhibitor and is applied in cosmetics, pharmaceuticals, and the like for purpose of prevention of oxidation or stabilization of medicine. The blending amount of nordihydroguaiaretic acid is preferably, about 0.01 % by weight or more, more preferably, about 0.1 % by weight or more, with respect to the total amount of the external preparation for skin. The blending amount of nordihydroguaiaretic acid is preferably, about 10 % by weight or less, more preferably, about 5 % by weight or less, with respect to the total amount of the external preparation for skin.

A chemical name of teprenone is geranylgeranylacetone. Teprenone has mucous membrane protecting and restoring action, cell growth-stimulating action, phospholipid synthesis-stimulating action and the like. The blending amount of teprenone is preferably, about 0.01 % by weight or more, more preferably, about 0.5 % by weight or more, more preferably, about 1.0 % by weight or more, with respect to the total amount of the external preparation for skin. The blending amount of teprenone is preferably, about 20 % by weight or less, more preferably, about 10 % by weight or less, more preferably, about 10 % by weight or less, with respect to the total amount of the external preparation for skin.

Allantoin has been used as a therapeutic for dermatological diseases and acts effectively in therapy of skin wound, prevention of skin roughening, and the like. The allantoin derivatives include dihydroxyaluminum allantoinate, chlorohydroxyaluminum allantoinate and the like. The blending amount of allantoin and the allantoin derivatives is preferably, about 0.01 % by weight or more, more preferably, about 0.1 % by weight or more, with respect to the total amount of the external preparation for skin. The blending amount of allantoin and the allantoin derivatives is preferably, about 5 % by weight or less, more preferably, about 3 % by weight or less, with respect to the total amount of the external preparation for skin.

The aminoethyl compounds are used for prevention and alleviation of skin roughening and alleviation of skin dullness, and are represented by NH₂CH₂CH₂X (X is -SO₂H or -SO₂SH). The blending amount of the aminoethyl compounds is preferably, about 0.0001 % by weight or more, more preferably, about 0.001 % by weight or more, with respect to the total amount of the external preparation for skin. The blending amount of the aminoethyl compounds is preferably, about 1.0 % by weight or less, and more preferably, about 0.3 % by weight or less, with respect to the total amount of the external preparation for skin is appropriate.

The alkylenediaminecarboxylic acid derivatives are used for improvement of the storage stability of external preparation for skins. They are particularly preferably ethylenediaminetetraacetic acid, the alkali-metal salts thereof such as Na, K, and Li salts, alkali-earth metal salts thereof such as Ca and Mg salts, ammonium salts thereof, alkanol salts thereof, and the like, and more preferably the Na salt. The blending amount of the alkylenediamine carboxylic acid derivatives is preferably, about 0.01 % by weight or more, more preferably, about 0.05 % by weight or more, with respect to the total amount of the external preparation for skin. The blending amount of the alkylenediamine carboxylic acid derivatives is preferably, about 0.5 % by weight or less, preferably, about 0.5 % by weight or less, with respect to the total amount of the external preparation for skin.

The betaine derivatives are used as a percutaneous accelerator, and are preferably alkyldimethylamino acids represented by General Formula 12, 2-alkyl-1-carboxymethyl-1-hydroxyethyl-2-imidazolines represented by General Formula 13, N-(3-acylaminopropyl)-N,N-dimethylaminoacetic acids represented by General Formula 14, and N-alkyl-N,N-dimethyl-3-amino-2-hydroxypropanesulfonic acids represented by General Formula 15.

An acylmethyltaurine is used as a percutaneous accelerator and is represented by General Formula 16.

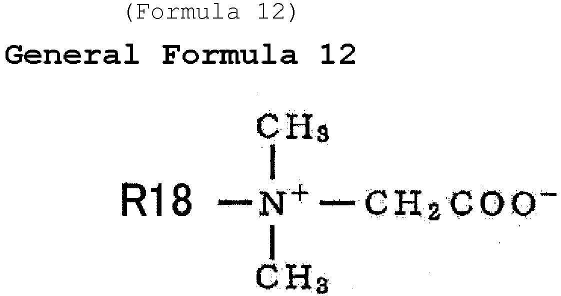

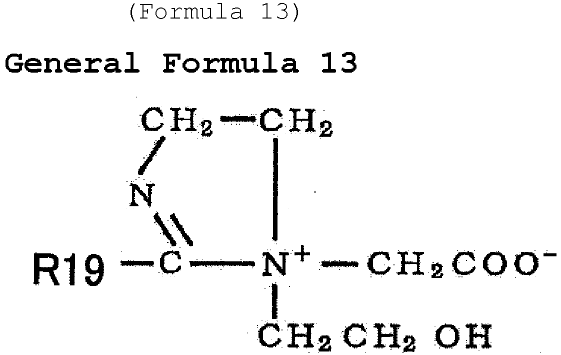

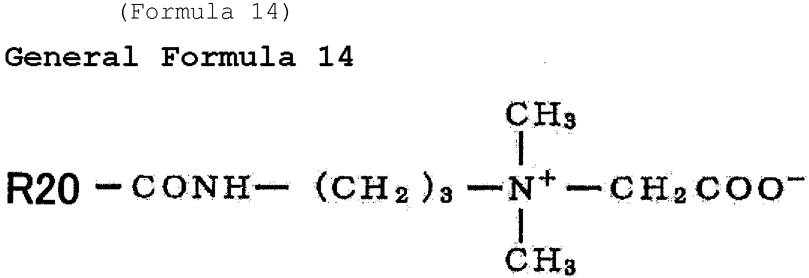

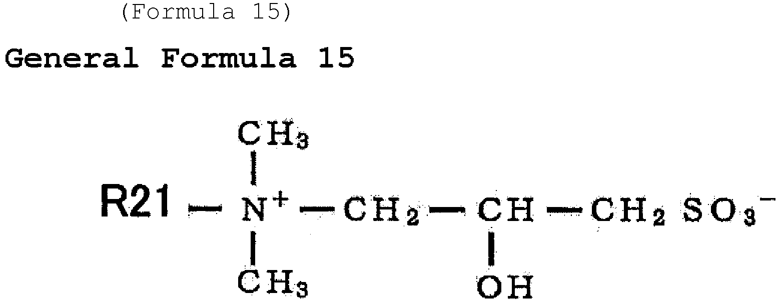

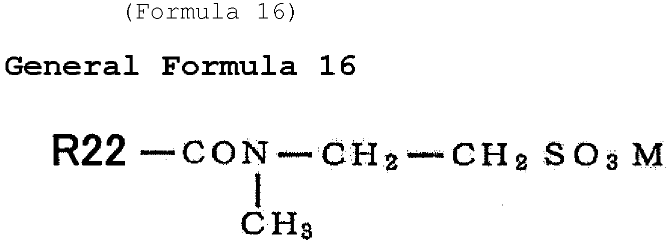

The total blended amount of the betaine derivatives and acylmethyltaurines is preferably, about 0.01 % by weight or more, more preferably, about 0.1 % by weight or more, with respect to the total amount of the external preparation for skin. The blending amount of total of the betaine derivatives and acylmethyltaurines is preferably, about 30 % by weight or less, preferably, about 20 % by weight or less, with respect to the total amount of the external preparation for skin.

Hederacosides include triterpenoid saponins obtained from the extracts of sapindus (Sapindus mukurossi Gaertn) and Akebia quinata Decne. The salts thereof include alkali-metal salts such as Na and K salts, ammonium salts, basic amino acid salts, alkanolamine salts, esters, and the like. Such an extract may be used as it is. The blending amount of hederacosides and the salts thereof is preferably, about 0.001 % by weight or more, more preferably, about 0.1 % by weight or more, with respect to the total amount of the external preparation for skin. The blending amount of hederacosides and the salts thereof is preferably, about 20 % by weight or less, more preferably, about 5 % by weight or less, with respect to the total amount of the external preparation for skin.

Gymnema saponins are triterpenoid saponins obtained from the extracts of Gymnema inodorum and asclepiadaceous Gymnema sylvestre R.Br. that is originally in India. The salts thereof include alkali-metal salts such as Na and K salts, ammonium salts, basic amino acid salts, alkanolamine salts, esters, and others. Such an extract may be used as it is. The blending amount of gymnema saponins is preferably, about 0.001 % by weight or more, more preferably, about 0.1 % by weight or more, with respect to the total amount of the external preparation for skin. The blending amount of gymnema saponins is preferably, about 20 % by weight or less, more preferably, about 5 % by weight or less, with respect to the total amount of the external preparation for skin.

Beet saponins are oleanolic acid glycosides obtained from the extract of chenopodiaceous sugar beet. The salts thereof include alkali-metal salts such as Na and K salts, ammonium salts, basic amino acid salts, alkanolamine salts, esters, and the like. Such an extract may be used as it is. The blending amount of beet saponins is preferably, about 0.001 % by weight or more, more preferably, about 0.1 % by weight or more, with respect to the total amount of the external preparation for skin. The blending amount of beet saponins is preferably, about 20 % by weight or less, more preferably, about 5 % by weight or less, with respect to the total amount of the external preparation for skin.

γ-pyrrone glycosides have action to prevent spot and freckles caused by sun burn. It is maltol-3-O-(6'-O-apiosyl)-glucosides or maltol-3-O-glucosides represented by General Formula 17. For example, those obtained from a pueraria root extract solution by column chromatography, preparative HPLC, thin layer chromatography or the like are used as γ-pyrrone glycosides. The blending amount of the γ-pyrrone glycosides is preferably, about 0.00001 % by weight or more, more preferably, about 0.0001 % by weight or more, with respect to the total amount of the external preparation for skin. The blending amount of the γ-pyrrone glycosides is preferably, about 2.5 % by weight or less, more preferably, about 1 % by weight or less, with respect to the total amount of the external preparation for skin. 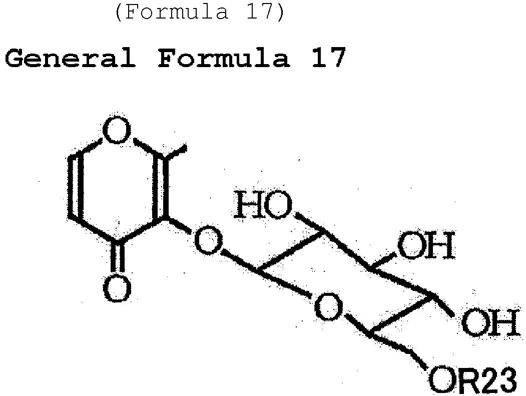 (wherein, R23 represents a hydrogen atom or

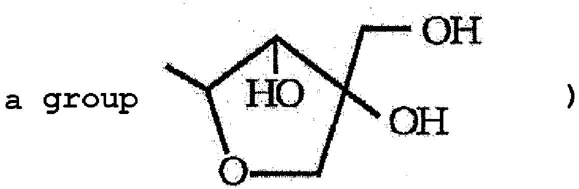

The biphenyl compounds have tyrosinase activity-inhibiting action and melanin-production suppressing action and are represented by General Formulae 18 and 19. Specifically, it includes dehydrodicreosol, dehydrodieugenol, tetrahydromagnolol and the like. The blending amount of the biphenyl compounds is preferably, about 0.0001 % by weight or more, more preferably, about 0.001 % by weight or more, with respect to the total amount of the external preparation for skin. The blending amount of the biphenyl compounds is preferably, about 20 % by weight or less, more preferably, about 5 % by weight or less, with respect to the total amount of the external preparation for skin.

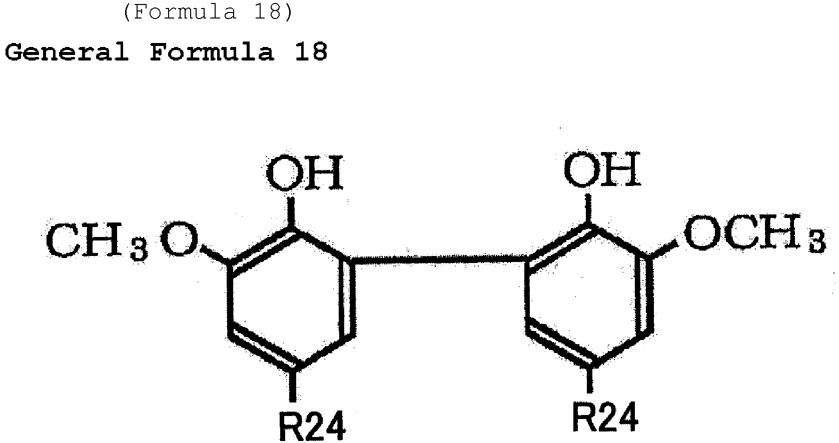

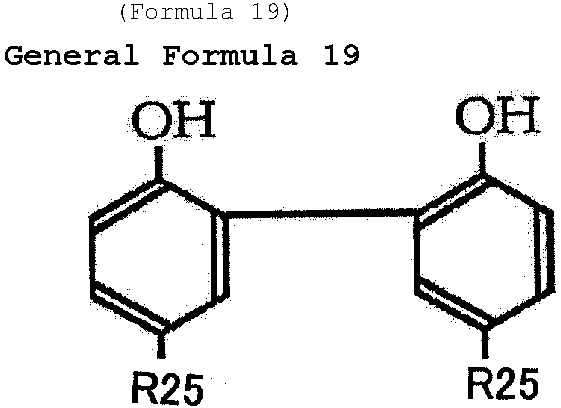

In Formulae 18 and 19, R24 is CH₃, C₂H₅, C₃H₇, CH₂OH, C₃H₆OH, or CH₂CH=CH₂; and R25 is a hydrogen atom or a straight or branched saturated hydrocarbon group having 1 to 8 carbons.

Sodium bisulfite is known as a substrate which is effective for stabilization of arbutin in external preparation for skins (Japanese Patent No. 2107858), and also has a similar action toward hydroquinone-α-D-glucose. The blending amount is adjusted to hydroquinone-α-D-glucose : sodium bisulfite be 1: 0.0001-1, preferably 1: 0.001-0.1 in weight ratio.

Fibronectin (cold-insoluble globulin) has an effect of improving the whitening action of the hydroquinone-α-D-glucose according to the present invention. The blending amount of fibronectin is preferably, about 0.000001 % by weight or more, preferably, about 0.1 % by weight or less, with respect to the total amount of the external preparation for skin.

Examples of plant extracts as a whitening agent include asparagus extract, marshmallow extract, snake-weed wort extract, artemisia capillaris flower extract, pea extract, rose fruit extract, scutellaria root extract, ononis extract, seaweed extract, firethorn fruit extract, licorice extract, bramble extract, sophorae root extract, raw sugar extract, Millettia reticulata or Mucuna birdwoodiana extract, acanthopanacis cortex extract, wheatgerm extract, asiasarum rootextract, crataegus extract, cassia nomame extract, peony extract, white lily extract, Inulabritannica flower extract, mulberry bark extract, soy bean extract, placenta extract, Aralia elafa extract, tea plant extract, Japanese angelica root extract, molasses extract, Rosa multiflora extract, Ampelopsis japonica extract, grape seed extract, beech extract, Flor de Manita extract, hop extract, Rosa rugosa flower extract, Chaenomeles lagenaria extract, Saxifraga stolonifera extract, coix seed extract, Momordicae grosvenori Swingle fruit extract, and the like. These extracts may be used alone or in combination of two or more as appropriately selected. The blending amount of the whitening components is preferably, about 0.01 % by weight or more, preferably, about 10 % by weight or less, with respect to the total amount of the external preparation for skin.

The phenol and the derivatives thereof used as whitening agent include hydroquinone glycosides, hydroquinone monoethyl ether, hydroquinone mono-n-propyl ether, hydroquinone mono-n-butyl ether, hydroquinone mono-n-hexadecyl ether, hydroquinone mono-n-octadecyl ether, p-ethylphenol, p-n-propylphenol, p-n-butylphenol, p-t-butylphenol, p-isopropylphenol, p-hexadecylphenol, p-octadecylphenol, 4-isopropylcatechol monobutyl ester, 4-isopropylcatechol monoheptadecyl ester, and the like. The blending amount of the phenol and the derivatives thereof is preferably, about 0.01 % by weight or more, more preferably, about 0.1 % by weight, with respect to the total amount of the external preparation for skin. The blending amount of the phenol and the derivatives thereof is preferably, about 20 % by weight or less, more preferably, about 10 % by weight or less, with respect to the total amount of the external preparation for skin.

### (2.3 Ultraviolet absorbent)

In the present specification, the term "ultraviolet absorbent" is also referred to as an organic ultraviolet absorbent and it means a compound that absorbs ultraviolet ray and converts it into and releases it as an infrared, visible, or other ray less harmful to the human body.

Any ultraviolet absorbents commonly used in conventional external preparation for skin may be used as the ultraviolet absorbents. The blending amount of the ultraviolet absorbents is preferably, about 0.01 % by weight or more, more preferably 0.5 % by weight or more, with respect to the total amount of the external preparation for skin. The blending amount of the ultraviolet absorbents is preferably, about 10 % by weight or less, preferably, about 8 % by weight or less, with respect to the total amount of the external preparation for skin.

Examples of the ultraviolet absorbent include and is selected from the followings: benzoic acid-based ultraviolet absorbents, anthranilic acid-based ultraviolet absorbents, salicylic acid-based ultraviolet absorbents, cinnamic acid-based ultraviolet absorbents, benzophenone-based ultraviolet absorbents, and other ultraviolet absorbent.

Examples of the benzoic acid-based ultraviolet absorbents include para-aminobenzoic acid (hereinafter, abbreviated as PABA), PABA monoglycerol ester, N,N-dipropoxy PABA ethyl ester, N,N-diethoxy PABA ethyl ester,N,N-dimethyl PABA ethyl ester, N,N-dimethyl PABA butyl ester, N,N-dimethyl PABA amyl ester, and N,N-dimethyl PABA octyl ester.

Examples of the anthranilic acid-based ultraviolet absorbents include homomenthyl-N-acetyl anthranilate.

Examples of the salicylic acid-based ultraviolet absorbents include amyl salicylate, menthyl salicylate, homomenthyl salicylate, octylsalicylate, phenyl salicylate, benzyl salicylate, and p-isopropanol phenyl salicylate.

Examples of the cinnamic acid-based ultraviolet absorbents include octyl cinnamate, ethyl-4-isopropyl cinnamate, methyl-2,5-diisopropyl cinnamate, ethyl-2,4-diisopropyl cinnamate, methyl-2,4-diisopropyl cinnamate,propyl-p-methoxycinnamate, isopropyl-p-methoxy cinnamate, isoamyl-p-methoxy cinnamate, isopropyl-p-methoxycinnamate, isoamyl-p-methoxycinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glyceryl mono-2-ethylhaxanoyl-diparamethoxy cinnamate.

Examples of the benzophenone-based ultraviolet absorbents include 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy4'-methylbenzophenone, 2-hydroxy-4-methoxybenzophenone-5-sulfonate salts, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone.

Examples of other ultraviolet absorbents include 3-(4'-methylbenzylidene)-d,l-camphor, 3-benzylidene-d,l-camphor, urocanic acid, ethyl urocanate ester, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t-butyl dibenzoylmethane, and 5-(3,3-dimethyl-2-norbornylidene)-3-pentan-2-one.

### (2.4 anti-inflammatory agent)

In the present specification, the term "anti-inflammatory agent" means a compound having a characteristic to suppress inflammatory process in a particular local area.

The blending amount of the anti-inflammatory agent varies according to the kind of the anti-inflammatory agent and cannot be specified definitely, but is preferably about 0.01 % by weight or more, preferably about 1 % by weight or less, with respect to the total amount of the external preparation for skin.

Examples of the anti-inflammatory agents include the followings: zinc oxide, sulfur and the derivatives thereof; glycyrrhizic acid, the derivatives and salts thereof, such as, glycyrrhizic acid, dipotassium glycyrrhizinate, monoammonium glycyrrhizinate; glycyrrhetic acid, the derivatives and salts thereof, such as, β-glycyrrhetic acid, stearyl glycyrrhetinate, and disodium 3-succinyloxyglycyrrhetinate; tranexamicacid, chondroitin sulfuric acid, mefenamic acid, phenylbutazone, indomethacin, ibuprofen, ketoprofen, allantoin, guaiazulene, the derivatives and salts thereof; and extracts of various microorganisms, plants and animals.

### (2.5 Cell-activating agent)

In the present specification, the term "cell-activating agent" means a substance having an action to accelerate cell growth.

The blending amount of the cell-activating agents varies according to the kind of the cell-activating agents, but is usually, 0.01 to 5 % by weight with respect to the external preparation for skin.

Examples of the cell-activating agents include the followings: CoQ10 deoxyribonucleic acid and the salts thereof; adenylic acid derivatives such as adenosine triphosphate and adenosine monophosphate, and the salts thereof; ribonucleic acid and the salts thereof; cyclic AMP's, cyclic GMP's, flavin adenine nucleotide, guanine, adenine, cytosine, thymine, xanthine and the derivatives thereof; caffeine, theophylline and the salts thereof; retinol and retinol derivatives such as retinol palmitate and retinol acetate; retinal derivatives such as retinal and dehydroretinal; carotenoids such as carotene and vitamin A's; thiamine and thiamine salts such as thiamine hydrochloride and thiamine sulfate; riboflavin and riboflavin salts such as riboflavin acetate; pyridoxine and pyridoxine salts such as, pyridoxine hydrochloride and pyridoxine dioctanoate; flavin adenine nucleotide; cyanocobalamin; folic acids; nicotinic acid and nicotinic acid derivatives such as nicotinic acid amide and benzyl nicotinate; vitamin B's such as cholines; γ-linolenic acid and the derivatives thereof; eicosapentaenoic acid and the derivatives thereof; estradiol and the derivatives and salts thereof; as well as organic acids such as glycolic acid, succinic acid, lactic acid and salicylic acid, the derivatives and salts thereof.

### (2.6 Antioxidant)

In the present specification, the term "antioxidant" means a component added for suppression of oxidation of the components in the product.

The content of the antioxidant varies according to the kind of antioxidant component and can not be specified definitely, but is preferably, about 0.01 % by weight or more, preferably about 10 % by weight or less, with respect to the total amount of the external preparation for skin. When an extract solution such as plant extract is used as it is, the content is an amount converted into dry solid matter.

Examples of the antioxidants include the followings: vitamin A's, the derivatives and salts thereof such as retinol, dehydroretinol, retinolacetate, retinolpalmitate, retinal, retinoic acid and vitamin A oil; carotenoids and the derivatives thereof such as α-carotene, β-carotene, γ-carotene, cryptoxanthin, astaxanthin, and fucoxanthin; vitamin B's, the derivatives and salts thereof such as pyridoxine, pyridoxal, pyridoxal-5-phosphate esters and pyridoxamine; vitamin C's, the derivatives and salts thereof such as ascorbic acid, sodium ascorbate, ascorbyl stearate, ascorbyl palmitate, ascorbyl dipalmitate and magnesium ascorbyl phosphate; vitamin D's such as ergocalciferol, cholecalciferol, and 1,2,5-dihydroxy-cholecalciferol and the derivatives and salts thereof; vitamin E's, the derivatives and salts thereof such as α-tocopherol, β-tocopherol, γ-tocopherol, δ-tocopherol, α-tocotrienol, β-tocotrienol, γ-tocotrienol, δ-tocotrienol, tocopherol acetate and tocopherol nicotinate; trolox, the derivatives and salts thereof; dihydroxytoluene, butylhydroxytoluene, butylhydroxyanisole, dibutylhydroxytoluene, α-lipoicacid, dehydrolipoic acid, glutathione, the derivatives and salts thereof; uric acid; erythorbic acids, the derivatives and salts thereof such as erythorbic acid and sodium erythorbate; gallic acids, the derivatives and salts thereof such as gallic acid and propyl gallate; rutin, the derivatives and salts thereof such as rutin and α-glycosyl-rutin; tryptophan and the derivatives and salts thereof; histidine, the derivatives and salts thereof; cysteine derivatives such as N-acetylcysteine, N-acetylhomocysteine, N-octanoylcysteine, and N-acetylcysteine methyl ester and the salts thereof; cystine derivatives such as N,N'-diacetylcystine dimethyl ester, N,N'-dioctanoylcystine dimethyl ester, and N,N'-dioctanoylhomocystine dimethyl ester and the salts thereof; carnosine, the derivatives and salts thereof; homocarnosine, the derivatives and salts thereof; anserine, the derivatives and salts thereof; carcinin, the derivatives and salts thereof; dipeptide or tripeptide derivatives containing histidine and/or tryptophan and/or histamine and the salts thereof; flavonoids such as flavanone, flavone, anthocyanin, anthocyanidin, flavonol, quercetin, quercitrin, myricetin, fisetin, hamamelis tannin, catechin, epicatechin, gallocatechin, epigallocatechin, epicatechin gallate, and epigallocatechin gallate; tannic acid, caffeic acid, ferulicacid, protocatechuic acid, chalcone, oryzanol, carnosol, sesamol, sesamine, sesamolin, gingerone, curcumin, tetrahydrocurcumin, clovamide, deoxyclovamide, shogaols, capsaicin, vanillylamide, ellagic acid, bromphenol, flavoglassine, melanoidin, riboflavin, riboflavin butyrate esters, flavin mononucleotide, flavin adenine nucleotide, ubiquinone, ubiquinol, mannitol, bilirubin, cholesterol, ebselene, selenomethionine, ceruloplasmin, transferrin, lactoferrin, albumin, bilirubin, superoxide dismutase, catalase, glutathione peroxidase, metallothionein, O-phosphono-pyridoxylidene rhodamine; as well as N-(2-hydroxybenzyl)amino acids, the derivatives and salts thereof and N-(4-pyridoxylmethylene)amino acids, the derivatives and salts thereof described in U.S. Patent No. 5,594,012.

Examples of the ascorbic acid, the derivatives and salts thereof to be used as an antioxidant include monoalkyl ascorbate esters such as ascorbyl monostearate, ascorbyl monopalmitate and ascorbyl monooleate; ascorbic acid monoester derivatives such as ascorbic acid monophosphate esters and ascorbyl-2-sulfate; ascorbic acid diester derivatives such as ascorbyl distearate, ascorbyl dipalmitate, ascorbyl dioleate and ascorbic acid diphosphate esters; ascorbic acid trialkyl esters such as ascorbyl tristearate, ascorbyltripalmitate, and ascorbyltrioleate; ascorbic acid triester derivatives such as ascorbic acid triphosphate ester, ascorbic acid glycosides such as ascorbyl-2-glucoside, and the like. In particular, L-ascorbic acid, commonly called vitamin C, has cell respiration action, enzyme-activating action, collagen-forming action by its strong reductive action and also melanin-reducing action. Regarding the blending amount, the effect appears when it is blended at 0.01 % by weight or more with respect to the total amount of the external preparation for skin, and the upper limit value is about 10 % by weight.

### (3. Other components)

In the external preparation for skin, in addition to the components described above, other components normally used in external preparation for skins of cosmetics and pharmaceuticals can be appropriately blended as needed. For example, such other additive components are lower alcohols, silicones, oils and fats, ester oils, sterols and the derivatives thereof, oily components such as hydrocarbons, oily substances, antioxidants, surfactants, moisturizing agents, wetting agents, perfumes, water, color materials, powders, drugs, chelating agents, pH adjusting agents, emulsifying agents, solubilizing agents, thickeners, gelling agents, preservatives, microbicides, acids and alkalis, ultraviolet absorbents, anti-inflammatory agents, whitening agents, solvents, keratolytic agents, antiphlogistics, refrigerants, astringents, macromolecule powders, hydroxy acids, vitamins and the derivatives thereof, saccharides and the derivatives thereof, organic acids, enzymes, inorganic powders, and the like. These components must be used in an amount and at a quality that does not impair the effects of the present invention.

### (4. External preparation for skin)

In the present specification, the term "external preparation for skin" means a preparation to be used for skin which accomplishes a desired effect when in contact with the skin. The present invention is particularly effective in applications continuously keeping contact with skin for an extended period of time (for example, an application continuously keeping contact with skin for 1 hour or more or an application continuously keeping contact with skin for 5 hours or more).

Apreferable example of the external preparation for skin is a cosmetic preparation.

Preferable examples of the cosmetic preparations include skincare cosmetic preparations. Specific examples of the cosmetic preparations include skincare cosmetic preparations such as skin lotion, emulsion, and cream; cosmetics such as foundation, eye shadow, lipstick, and rouge for cheek; hair cosmetic preparations, emollient cream, emollient lotion, cream, cream rinse, cold cream, vanishing cream, lotion, facial mask, gel, face pack, soap, body soap, shampoo, conditioner, rinse, face wash, shaving cream, hair cream, hair lotion, hair treatment, hair pack, gloss, lip cream, cake, and the like. The present invention is particularly effective in applications in which moisturizing effect is desired. For example, the present invention is effective as a skincare cosmetic preparation. The invention is particularly effective in applications contacting with the skin for an extended period of time, but also effective in applications such as face wash and shampoo where it is washed away after use for a short period of time.

As described above, cosmetics are also included in the cosmetic preparations. Cosmetics are classified into cosmetics for cleaning, cosmetics for hair, basic cosmetics, makeup cosmetics, fragrant cosmetics, cosmetics for sun-burn, cosmetics for anti-sunburn, nail cosmetics, eye liner cosmetics, eye shadow cosmetics, rouge for cheek/lip cosmetics, oral cavity cosmetics, and the like. The present invention is effective in any application of them.

The external preparation for skin may be a drug or a quasi-drug. For example, the phosphorylated saccharide may be blended to an ointment containing a pharmaceutically effective component.

Preferable examples of the external preparation for skin include emulsion, skin lotion, cream, shampoo, rinse, conditioner, face wash, shaving cream, lotion, cleansing oil, body soap, soap, gel, face pack, lipstick, gloss, lip cream, foundation, eye shadow, rouge for cheek, hair pack, anti-sunburn cosmetics, hair cosmetic preparation and oral cavity cosmetics.

Blending of a phosphorylated saccharide (in particular, inorganic salt of phosphorylated saccharide) to an external preparation for skin (for example, cosmetic or quasi drug such as emulsion, skin lotion, cream, shampoo, or face wash) results in an external preparation for skin that increases skin moisturization and alleviates symptoms such as dry skin, skin roughening, allergy, and atopic dermatitis. The external preparation for skin disclosed herein increases skin moisturization and thus, activates skin metabolism. Further, the external preparation for skin disclosed herein is effective in removing melanin dye and active oxygen generated by ultraviolet irradiation rapidly, thus exhibiting whitening effect, and preventing skin damage. The external preparation for skin disclosed herein also has an effect to improve the barrier function of the skin. Therefore, the external preparation for skin containing the phosphorylated saccharide alleviates adverse effect on the skin caused by drying and ultraviolet ray, improves dyschromatosis such as spots or freckles, and delays aging phenomena such as dullness, wrinkles, sag, and alopecia, and additionally, it is effective to pimple, chilblain, rash, and miliaria.

Examples of the dosage forms of the external preparation for skin disclosed herein include ointment, thickened gel system, lotion, water in oil emulsion, oil in water emulsion, solid, sheet, powder, gel, mousse and spray. The external preparation for skin may be a product in the shape of sheet impregnated with the preparation such as makeup removing facial mask.

When the dosage form of the external preparation for skin is lotion, emulsion, or thickening gel sysem, in terms of improvement of its effect, it is preferable to blend among the components above, in particular among the thickeners, a water-soluble thickener, for example, a plant-derived macromolecule such as gum arabic, tragacanth gum, galactan, guar gum, carrageenan, pectin, quince seed (Cydonia oblonga) extract, or brown algae powder; a microbial-derived macromolecule such as xanthan gum, dextran, or pullulan; an animal-derived macromolecule such as collagen, casein, albumin, or gelatin; a starch such as carboxymethyl starch or methylhydroxy starch; a cellulose such as methyl cellulose, nitrocellulose, ethyl cellulose, methylhydroxypropyl cellulose hydroxyethyl cellulose, cellulose sulfate salt, hydroxypropyl cellulose, carboxymethyl cellulose, crystalline cellulose, or cellulose powder; a vinyl-type macromolecule such as polyvinyl alcohol, polyvinyl methyl ether, polyvinyl pyrrolidone or carboxy vinyl polymer; an acrylic-type polymer such as polyacrylic acid or the salt thereof or polyacrylimide; an organic thickener such as glycyrrhizic acid or alginic acid or an inorganic thickener such as bentonite, hectolite, labonite, magnesium aluminum silicate, or silicic anhydride; in combination with a lower alcohol such as ethanol or isopropanol among the alcohols. The blending amount of the water-soluble thickener is 0.01 to 5% by weight, preferably 0.1 to 3 % by weight, with respect to the total amount of the external preparation for skin. The blending amount of the lower alcohol is 0.3 to 35 % by weight with respect to the total amount of the external preparation for skin. It is preferable that the blending ratio of the phosphorylated saccharide to the lower alcohol in the external preparation for skin is 3:1 to 1:3 (by weight) .

### (5. Production method of external preparation for skin)

The external preparation for skin disclosed herein can be produced by a known method.

### (6. Usage of external preparation for skin)

The external preparation for skin disclosed herein can be used by a method similar to the conventionally used methods . The external preparation for skin disclosed herein can be applied on the skin in arbitrary frequency, for example, once a day, twice a day, three times a day, or the like. The application area where the external preparation for skin disclosed herein is to be applied is arbitrary. It can be selected appropriately according to the kind of the external preparation for skin. The external preparation for skin disclosed herein may be applied on any area of the skin of the body. Examples of the areas applied include face, head, arm, hand, leg and foot.

### EXAMPLES

The phosphorylated saccharide used in the following Examples and Test Example refers to the phosphorylated saccharide prepared from potato starch described in Japanese Laid-open Patent Publication No. 8-104696, Example 1. In other words, it is a mixture of phosphorylated saccharides in which oligosaccharides of 2 to 8 α-1,4-bound glucoses bound to one to two phosphate groups in the molecule. The phosphorylated saccharide is a mixture of phosphorylated saccharide in which oligosaccharides of 3, 4 and 5 glucoses bound to one phosphate group in the molecule and phosphorylated saccharide in which oligosaccharides of 5, 6, 7 and 8 glucoses bound to two phosphate groups in the molecule. Wherein the molar ratio of the phosphorylated saccharide having one phosphate group bounded to phosphorylated saccharide having two phosphate groups bounded is about 8:2.

The reduced phosphorylated saccharide used in the following Examples was prepared by a method which hydrogenates the phosphorylated saccharide in the presence of a nickel catalyst under high temperature and high pressure . In the laboratory-scale experiment, it can be prepared easily by coexisting it with sodium borohydride at room temperature for about 1 hour. Reduction of the reducing terminal can be generally confirmed easily by equimolar generation of sorbitol to the phosphorylated saccharide after acid hydrolysis of the phosphorylated saccharide (Kamasaka, H., To-o, K., Kusaka, K., Kuriki, T., Kometani, T., Hayashi, H., and Okada, S., Biosci. Biotech. Biochem. 61, 238-244, 1997) .

Moreover, the phosphate group in the phosphorylated saccharide and the reduced phosphorylated saccharide can be prepared to be a calcium, magnesium, potassium, sodium, or zinc salt. Regarding the preparation method of the sodium salt, it can be prepared by the method described in Japanese Laid-open Patent Publication No. 8-104696, Example 1. Furthermore, the calcium salt can be prepared using calcium chloride in place of sodium chloride in this Example. The magnesium salt can be prepared using magnesium chloride in place of sodium chloride in this Example . The potassium salt can be prepared using potassium chloride in place of sodium chloride in this Example . The zinc salt can be prepared using zinc chloride in place of sodium chloride in this Example. The salts thus prepared were used in the following Examples and Test Examples.

In addition to this method using an ion-exchange resin, various metal salts of the phosphorylated saccharide can be prepared by desalination using general electrodialysis and subsequent addition of each metal salt. It should be noted that phosphorylated saccharide calcium salt which is sold from Ezaki Glico Co., Ltd. (Osaka) as phosphorylated oligosaccharide calcium salt can be used preferably.

### (Example 1: Moisturizing effect attained by skin lotion containing phosphorylated saccharide potassium salt)

A skin lotion containing 0.5% phosphorylated saccharide potassium salt, 2.5% glycerol, 1.0% polyoxyethylene sorbitan monolaurate, a preservative, a perfume and purified water was prepared. Separately, the same skin lotion except for not containing phosphorylated saccharide potassium salt was also prepared as the control. These skin lotions were used by 16 women aged 22 to 34 year old for 2 months. After that, the effectiveness thereof was researched. Skin trouble such as skin reddening was not occurred upon use of it for all women. In addition, 87.5% of the subjects answered that they felt more moisture with the skin lotion containing the phosphorylated saccharide potassium salt.

### (Example 2: moisturizing effect attained by cream containing phosphorylated saccharide potassium salt)

A cosmetic cream consisting of 0.1% of phosphorylated saccharide potassium salt, 0.2% phosphorylated saccharide calcium salt, 2% beeswax, 5.0% stearyl alcohol, 10.0% squalane, 2.5% glycerol monostearate, 1.0% polyoxyethylene cetyl ether, 5.0% glycerol, 0.2% potassium hydroxide, an antioxidant and purified water was prepared. The cream was used by 10 women aged 22 to 34 year old for 2 weeks. After that, impressions of use were heard from them. As a result, there was no subject whose skin troubles such as skin roughening become worse. In addition, 80% of the subjects felt moisture.

### (Example 3: moisturizing effect attained by cream containing phosphorylated saccharide potassium salt)

A cosmetic cream consisting of 0.2% phosphorylated saccharide potassium salt, 0.2% phosphorylated saccharide calciumsalt, 0.2% phosphorylated saccharide magnesium salt, 2% beeswax, 5.0% stearyl alcohol, 10.0% squalane, 2.5% glycerol monostearate, 1.0% polyoxyethylene cetyl ether, 5.0% glycerol, 0.2% potassium hydroxide, an antioxidant and purified water was prepared. The cream was used by 10 women aged 22 to 34 year old for 2 weeks. After that, impressions of use were heard from them. As a result, there was no subject whose skin troubles such as skin roughening become worse. In addition, 90% of the subjects felt moisture.

### (Example 4: influence of phosphorylated saccharide calcium salt on collagen production by human skin fibroblast)

Normal human skin fibroblast cells were adjusted with 10% D-MEM (manufactured by Invitrogen) to a density of 2.5×10⁴ cells per well, and preincubated on the 96-well plate for 24 hours. After removal of the medium, phosphorylated saccharide calcium salt adjusted to a concentration of 1 % with 4% D-MEM was added to each well as a test sample, and then the mixture was cultured at 37°C under 5% CO₂ for 72 hours. After the completion of the culture, the amount of collagen in the medium was measured. The amount of collagen was measured by using a Sircol collagen assay Kit (manufactured by Biocolor Ltd.). No generation of collagen was observed in the media without added phosphorylated saccharide calcium salt. However, the amount of collagen in the medium to which 1% phosphorylated saccharide calcium salt was added was 11.9 µg/ml, and thus significant collagen induction was observed. Further, no induction of collagen production was observed when calcium chloride was used.

### (Example 5: influence of phosphorylated saccharide calcium salt and ascorbic acid on collagen production by human skin fibroblast)

Normal human skin fibroblast cells were adjusted with 10% D-MEM (manufactured by Invitrogen) to a density of 2.5×10⁴ cells per well, and preincubated on the 96-well plate for 24 hours. After removal of the medium, phosphorylated saccharide calcium salt adjusted to a concentration of 1 % with 4% D-MEM and 0.0044% ascorbic acid as a test sample, or 0.0044% ascorbic acid as a control was added to each well, and then the mixture was cultured at 37°C under 5% CO₂ for 72 hours. After the completion of the culture, the amounts of collagen in the medium and on the cell surface were measured. The amount of collagen was measured by using a Sircol collagen assay Kit (manufactured by Biocolor Ltd.). The collagen on cell surface was measured after pepsin treatment. The amounts of collagen in the media and on the cell surface in the wells to which 1% phosphorylated saccharide calcium salt and 0.0044% ascorbic acid were added were respectively 95.3 µg/ml and 31.4 µg/ml. The amounts of collagen in the medium and on the cell surface of control to which only ascorbic acid was added were respectively 55.1 µg/ml and 23.1 µg/ml. In the samples using the combination of phosphorylated saccharide calcium salt and ascorbic acid, a high collagen-inducing effect was observed. The effect was about 173% in the medium and 136% on the cell surface compared to those attained by using ascorbic acid alone.

### (Example 6: influence of phosphorylated saccharide potassium salt on collagen production by human skin fibroblast)

Normal human skin fibroblast cells were adjusted with 10% D-MEM (manufactured by Invitrogen) to a density of 2.5×10⁴ cells per well, and preincubated on the 96-well plate for 24 hours. After removal of the medium, phosphorylated saccharide potassium salt adjusted to a concentration of 1 % with 4% D-MEM was added to each well as a test sample, and then cultured at 37°C under 5% CO₂ for 72 hours. After the completion of the culture, the amount of collagen in the medium was measured. The amount of collagen was measured by using a Sircol collagen assay Kit (manufactured by Biocolor Ltd.) . No generation of collagen was observed in the media without added phosphorylated saccharide potassium salt. However, the amount of collagen in the medium to which 1% phosphorylated saccharide potassium salt was added was 40.1 µg/ml, and thus significant collagen induction was observed. Further, induction of the collagen production by potassium chloride was as low as 2.2 µg/ml.

### (Example 7: influence of phosphorylated saccharide potassium salt and ascorbic acid on collagen production by human skin fibroblast)

Normal human skin fibroblast cells were adjusted with 10% D-MEM (manufactured by Invitrogen) to a density of 2.5×10⁴ cells per well, and preincubated on the 96-well plate for 24 hours. After removal of the medium, phosphorylated saccharide potassium salt adjusted to a concentration of 1 % with 4% D-MEM and 0.0044% ascorbic acid as a test sample, or 0.0044% ascorbic acid as control was added to each well, and then the mixture was cultured at 37°C under 5% CO₂ for 72 hours. After the completion of the culture, the amounts of collagen in the medium and the cell surface were measured. The amount of collagen was measured by using a Sircol collagen assay Kit (manufactured by Biocolor Ltd.). The collagen on cell surface was measured after pepsin treatment. The amounts of collagen in the media and on the cell surface for wells to which 1% phosphorylated saccharide potassium salt and 0.0044% ascorbic acid were added were respectively 119.8 µg/ml and 23.3 µg/ml. The amounts of collagen in the medium and on the cell surface for control to which only ascorbic acid was added were respectively 55.1 µg/ml and 23.1 µg/ml. In the samples using the combination of phosphorylated saccharide potassium salt and ascorbic acid, a collagen-inducing effect was observed. The effect was about 217% in the medium and 100% on the cell surface compared to those attained by using ascorbic acid alone.

### (Example 8: influence of phosphorylated saccharide mineral salt on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, four kinds of samples (phosphorylated saccharide calcium salt adjusted to a concentration of 1 % with 4% D-MEM, phosphorylated saccharide magnesium salt adjusted to a concentration of 1% with 4% D-MEM, a mixture of phosphorylated saccharide calcium salt and phosphorylated saccharide magnesium salt adjusted to a concentration of 1% with 4% D-MEM, and ascorbic acid adjusted to 0.0044% with 4% D-MEM) were added to each well as test samples, and then cultured at 37°C under 5% CO₂ for 72 hours. After the completion of the culture, the amounts of collagen in the medium and on the cell surface were measured. The amount of collagen was measured by using a Sircol collagen assay Kit (manufactured by Biocolor Ltd.) . The amount of collagen in the media to which 1% phosphorylated saccharide calcium salt was added was 56.25 µg/ml. The amount of collagen in the media to which phosphorylated saccharide magnesium salt was added was 61.86 µg/ml. The amount of collagen in the media to which a mixture of 1% phosphorylated saccharide calcium salt and phosphorylated saccharide magnesium salt was added was 107.0 µg/ml. The amount of collagen in the media to which ascorbic acid was added was 44.1 µg/ml. Almost no collagen production was observed in the media without the addtion. From these results, it was confirmed that combined use of phosphorylated oligosaccharide calcium salt and phosphorylated oligosaccharide magnesium salt induced additively collagen-inducing effect without reducing their effect. Further, the effect of the mixture was significantly greater than the induction effect by ascorbic acid.

### (Example 9: influence of reduced phosphorylated saccharide calcium salt and ascorbic acid on the collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, reduced phosphorylated saccharide calcium salt adjusted to a concentration of 1 % with 4% D-MEM and 0.0044% ascorbic acid as a test sample, or 0.0044% ascorbic acid as a control was added to each well, and then the mixture was cultured at 37°C under 5% CO₂ for 72 hours. After the completion of the culture, the amounts of collagen in the medium and on the cell surface were measured. The amount of collagen was measured by using a Sircol collagen assay Kit (manufactured by Biocolor Ltd.). The collagen on cell surface was measured after pepsin treatment. The amounts of collagen in the media and on cell surface in the wells to which 1% reduced phosphorylated saccharide calcium salt and 0.0044% ascorbic acid were added were respectively 89.2 µg/ml and 23.4 µg/ml. The amounts of collagen in the medium and on the cell surface of the control to which only ascorbic acid was added were respectively 50.1 µg/ml and 23.0 µg/ml. In the samples using the combination of reduced phosphorylated saccharide calcium salt and ascorbic acid, a high collagen-inducing effect was observed. The effect was about 178% in the medium compared to those attained by using ascorbic acid alone.

### (Example 10: influence of reduced phosphorylated saccharide magnesium salt and ascorbic acid on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, reduced phosphorylated saccharide magnesium salt were adjusted to a concentration of 1 % with 4% D-MEM and 0.0044% ascorbic acid as a test sample, or 0.0044% ascorbic acid as control was added to each well, and then the mixture was cultured at 37°C under 5% CO₂ for 72 hours. After the completion of the culture, the amounts of collagen in the medium and on the cell surface were measured. The amount of collagen was measured by using a Sircol collagen assay Kit (manufactured by Biocolor Ltd.). The collagen on cell surface was measured after pepsin treatment. The amounts of collagen in the media and on cell surface in the wells to which 1% reduced phosphorylated saccharide magnesium salt and 0.0044% ascorbic acid were added were respectively 95.3 µg/ml and 20.5 µg/ml. The amounts of collagen in the medium and on the cell surface of the control to which only ascorbic acid was added were respectively 52.1 µg/ml and 21.3 µg/ml. In the samples using the combination of phosphorylated saccharide magnesium salt and ascorbic acid, a high collagen-inducing effect was observed. The effect was about 183% in the medium compared to those attained by using ascorbic acid alone.

### (Example 11)

A cosmetic cream consisting of 0.5% phosphorylated saccharide potassium salt, 0.5% phosphorylated saccharide magnesium salt, 2% beeswax, 5.0% stearyl alcohol, 10.0% squalane, 2.5% glycerol monostearate, 1.0% polyoxyethylene cetyl ether, 5.0% glycerol, 0.2% potassium hydroxide, an antioxidant and purified water was prepared. The cream was used by 10 women aged 25 to 35 year old for 2 weeks. After that, impressions of use were heard from them. As a result, there was no subject whose skin troubles such as skin roughening become worse. In addition, 88% of the subjects felt moisture.

### (Example 12)

A cosmetic cream consisting of 0.5% phosphorylated saccharide magnesium salt, 0.5% phosphorylated saccharide sodium salt, 2% beeswax, 5.0% stearyl alcohol, 10.0% squalane, 2.5%glycerol monostearate, 1.0%polyoxyethylene cetyl ether, 5.0% glycerol, 0.2% potassium hydroxide, an antioxidant and purified water was prepared. The cream was used by 10 women aged 25 to 35 year old for 2 weeks. After that, impressions of use were heard from them. As a result, there was no subject whose skin trouble such as skin roughening become worth. In addition, 84% of the subjects felt moisture.

### (Example 13: influence of phosphorylated saccharide magnesium salt on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, phosphorylated saccharide magnesium salt adjusted to a concentration of 1 % with 4% D-MEM was added to each well as a test sample, and cultured at 37°C under 5% CO₂ for 72 hours. After the completion of the culture, the amount of collagen in the medium was measured. The amount of collagen was measured by using a Sircol collagen assay Kit (manufactured by Biocolor Ltd.) . No generation of collagen was observed in the media without added phosphorylated saccharide magnesium salt. However, the amount of collagen in the medium to which 1% phosphorylated saccharide magnesium salt was added was 19.7 µg/ml, and thus significant collagen induction was observed. Further, no significant collagen production induction was observed when magnesium chloride was used.

### (Example 14: influence of phosphorylated saccharide magnesium salt and ascorbic acid on collagen induction by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, phosphorylated saccharide magnesium salt adjusted to a concentration of 1 % with 4% D-MEM and 0.0044% ascorbic acid as a test sample or 0.0044% ascorbic acid as a control was added to each well, and then the mixture was cultured at 37°C under 5% CO₂ for 72 hours. After the completion of the culture, the amounts of collagen in the medium and on the cell surface were measured. The amount of collagen was measured by using a Sircol collagen assay Kit (manufactured by Biocolor Ltd.). The collagen of cell surface was measured after pepsin treatment. The amounts of collagen in the media and on cell surface in the wells to which 1% phosphorylated saccharide magnesium salt and 0.0044% ascorbic acid were added were respectively 83.9 µg/ml and 20.3 µg/ml. The amounts of collagen in the medium and on the cell surface of the control to which only ascorbic acid was added were respectively 55.1 µg/ml and 23.1 µg/ml. In the samples using the combination of phosphorylated saccharide calcium salt and ascorbic acid, a collagen-inducing effect was observed. The effect was about 152% in the medium compared to those attained by using ascorbic acid alone.

### (Example 15: influence of reduced phosphorylated saccharide calcium salt on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, reduced phosphorylated saccharide calcium salt adjusted to a concentration of 1 % with 4% D-MEM was added to each well as a test sample, and cultured at 37°C under 5% CO₂ for 72 hours. After the completion of the culture, the amount of collagen in the medium was measured. The amount of collagen was measured by using a Sircol collagen assay Kit (manufactured by Biocolor Ltd.). There was no generation of collagen observed in the media to which no reduced phosphorylated saccharide calcium salt was added. However, the amount of collagen in the medium to which 1% phosphorylated saccharide calcium salt was added was 29.7 µg/ml, and thus significant collagen induction was observed.

### (Example 16: influence of reduced phosphorylated saccharide potassium salt on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. Then, reduced phosphorylated saccharide potassium salt adjusted to a concentration of 1 % with 4% D-MEM was added to each well as a test sample, and then cultured at 37°C under 5% CO₂ for 72 hours. After the completion of the culture, the amount of collagen in the medium was measured. The amount of collagen was measured by using a Sircol collagen assay Kit (manufactured by Biocolor Ltd.). There was no generation of collagen observed in the media to which no reduced phosphorylated saccharide potassium salt was added. However, the amount of collagen in the medium to which 1% reduced phosphorylated saccharide potassium salt was added was 24.1 µg/ml, and thus significant collagen induction was observed.

### (Example 17: influence of reduced phosphorylated saccharide magnesium salt on collagen production by human skin fibroblast)

Normal human skin fibroblast cells were adjusted with 10% D-MEM (manufactured by Invitrogen) to a density of 2.5×10⁴ cells per well, and then preincubated on the 96-well plate for 24 hours. After removal of the medium, reduced phosphorylated saccharide magnesium salt adjusted to a concentration of 1 % with 4% D-MEM was added to each well as a test sample, and then cultured at 37°C under 5% CO₂ for 72 hours. After the completion of the culture, the amount of collagen in the medium was measured. The amount of collagen was measured by using a Sircol collagen assay Kit (manufactured by Biocolor Ltd.). There was no generation of collagen observed in the media to which no reduced phosphorylated saccharide magnesium salt was added. However, the amount of collagen in the medium to which 1% reduced phosphorylated saccharide magnesium salt was added was 29.9 µg/ml, and thus significant collagen induction was observed.

### (Example 18: influence of phosphorylated saccharide calcium salt on collagen induction by human fibroblast)

Human fibroblast cells were adjusted with 10% D-MEM (manufactured by Dulbecco) to a density of 2.5×10⁴ cells per well, and then preincubated on the 96-well plate for 24 hours . Then, phosphorylated saccharide calcium salt adjusted to a concentration of 0.1 % with 4% D-MEM was added to each well as a test sample, and then cultured at 37°C for 72 hours. After the completion of the culture, the amount of collagen in the medium was measured. The amount of collagen was measured by using a Sircol collagen assay Kit (manufactured by Biocolor Ltd.). The collagen produced in medium to which no phosphorylated saccharide calcium salt was added was 4.6 µg/ml. However, the amount of collagen in the medium to which 0.1% phosphorylated saccharide calcium salt was added was 17.8 µg/ml, and thus significant collagen induction was observed.

### (Example 19: water-retention effect by phosphorylated saccharide mineral salt)

0.1% to 1% Aqueous phosphorylated saccharide mineral salt solutions were prepared. Ten µl/paper of the aqueous phosphorylated saccharide mineral salt solution was impregnated into a paper disc (8 mm) [manufactured by Advantech], and the water-retention effect was evaluated by measuring the remaining water over time for 10 minutes under a constant condition. For comparison, 0.1% solution of hyaluronic acid or 1% solution of glycerol, which are known as water retention component, were used. Distilled water was used as a control. The results obtained are shown in the following Table. It was confirmed that all phosphorylated saccharide mineral salts had a water-retention effect greater than that of control. In addition, phosphorylated saccharide magnesium salt and phosphorylated saccharide calcium salt had a water-retention effect higher than those of the 0.1% hyaluronic acid solution or 1% glycerol solution. Results are shown in Table 1A.

**(Table 1A)**

| Water retention ratio (%) | | | |
|---|---|---|---|
| **Time (min)** | **Concentration (%)** | **Remaining ratio after 1 minute (%)** | **Remaining ratio after 10 minutes (%)** |
| **glycerol** | **1.0** | **98.84** | **88.23** |
| **hyaluronic acid** | **0.1** | **98.84** | **83.73** |
| **distilled water** | **-** | **98.84** | **73.28** |
| **potassium salt of phosphorylated saccharide** | **0.1** | **98.84** | **74.12** |
| **potassium salt of phosphorylated saccharide** | **1.0** | **98.84** | **78.18** |
| **magnesium salt of phosphorylated saccharide** | **0.1** | **94.26** | **80.16** |
| **magnesium salt of phosphorylated saccharide** | **1.0** | **94.26** | **90.49** |
| **calcium salt of phosphorylated saccharide** | **0.1** | **94.26** | **81.46** |
| **calcium salt of phosphorylated saccharide** | **1.0** | **97.14** | **90.53** |
| **sodium salt of phosphorylated saccharide** | **1.0** | **96.72** | **82.22** |

### (Example 20: skin lotion)

According to the following formulation, a skin lotion was prepared by a conventional method.

**(Table 1)**

| Skin lotion composition | | |
|---|---|---|
| **Component Name** | **Function** | **Blending amount (% by weight)** |
| **Propylene glycol** | **Moisturizing agent** | **5.0** |
| **Ethanol** | | **14.0** |
| **POE (20) oleyl ether** | | **0.5** |
| **Sodium 2-hydroxy-4-methoxybenzophe none-5-sulfonate** | **Ultraviolet absorbing agent** | **0.1** |
| **Methylparaben** | | **0.1** |
| **Citric acid** | | **0.01** |
| **Sodium citrate** | | **0.1** |
| **Water-soluble placenta extract^{*1}** | **Moisturizing agent** | **2.0** |
| **Sodium hyaluronate** | **Moisturizing agent** | **0.3** |
| **Reduced phosphorylated saccharide magnesium salt** | | **5.0** |
| **Perfume** | | **0.05** |
| **Preservative** | | **Appropriate amount** |
| **Ion-exchanged water** | | **Remaining amount** |

| | | |
|---|---|---|
| ^{*1} Swine derived Pharconix BPS (manufactured by Ichimaru Pharcos Co., Ltd.) was used as the water-soluble placenta extract. | | |

Aforementioned skin lotion of the present invention was applied to the faces of half of the volunteer subjects (4 men and 6 women, total 10 persons, aged 27 to 47 year old), while a comparative skin lotion without the phosphorylated saccharide was applied to the same site of the remaining other half subjects. The ten subjects applied repeatedly the skin lotion in an appropriate amount twice a day for 14 days. 30 days after the completion of the test above, the subjects were crossed over each other, and the same test was carried out at the site different from the previous site to keep the equality of the both of the test groups. The test was controlled under double-blinded.

As a result, eight out of ten subjects answered that the skin lotion of the Example had far superior moisturization feeling and feelings of the alleviation of wrinkles and sag compared with those containing no phosphorylated saccharide.

### (Example 21: skin lotion)

According to the following formulation, a skin lotion was prepared by a conventional method.

**(Table 2)**

| Skin lotion composition | | |
|---|---|---|
| **Component name** | **Function** | **Blending amount (% by weight)** |
| **1,3-Butylene glycol** | **Moisturizing agent** | **3.0** |
| **Polyoxyethylene (20) sorbitan monolaurate** | | **1.0** |
| **Sorbitol (70%)** | **Moisturizing agent** | **2.0** |
| **Sodium pyrrolidone carboxylate solution** | | **3.0** |
| **Ethanol** | | **10** |
| **Magnesium ascorbyl phosphate** | **Moisturizing agent** | **0.5** |
| **Phosphorylated saccharide magnesium salt** | | **5.0** |
| **Perfume** | | **0.05** |
| **Preservative** | | **Appropriate amount** |
| **Ion-exchanged water** | | **Remaining amount** |

Aforementioned skin lotion of the present invention was applied to the faces of half of the volunteer subjects (5 men and 5 women, total 10 persons, aged 29 to 38 year old), while a comparative skin lotion without the phosphorylated saccharide was applied to the same site of the remaining other half subjects. The 10 subjects applied repeatedly the skin lotion in an appropriate amount twice a day for 30 days. 30 days after the completion of the test above, the subjects were crossed over each other, and the same test was carried out at the site different from the previous site to keep the equality of the both of the test groups. The test was controlled under double-blinded.

As a result, eight out of ten subjects answered that the skin lotion of the Example had superior moisturization feeling and feelings of the alleviation of wrinkles and sag compared with those containing no phosphorylated saccharide.

### (Example 22: Skin lotion)

According to the following formulation, a skin lotion was prepared by a conventional method.

**(Table 3)**

| Skin lotion composition | | |
|---|---|---|
| **Component name** | **Function** | **Blending amount (% by weight)** |
| **Glycerol** | **Moisturizing agent** | **5.0** |
| **Propylene glycol** | | **4.0** |
| **Ethanol** | | **8.0** |
| **POE (20) oleyl ether** | | **0.5** |
| **Allantoin** | **Anti-inflammatory agent (whitening component)** | **0.1** |
| **Citric acid** | | **0.01** |
| **Na citrate** | | **0.1** |
| **Acerola extract ^{*1}** | **Moisturizing agent** | **2.0** |
| **Sodium hyaluronate** | **Moisturizing agent** | **2.0** |
| **Phosphorylated saccharide potassium salt** | | **5.0** |
| **Perfume** | | **0.05** |
| **Preservative** | | **Appropriate amount** |
| **Ion-exchanged water** | | **Remaining amount** |

| | | |
|---|---|---|
| ^{*1} Nichirei Acerola Extract WB (manufactured by Nichirei Corporation) was used as the acerola extract. | | |

Aforementioned skin lotion of the present invention was applied to the faces of half of the volunteer subjects (5 men and 5 women, total 10 persons, aged 30 to 39 year old), while a comparative skin lotion without the phosphorylated saccharide was applied to the same site of the remaining other half subjects. The 10 subjects applied repeatedly the skin lotion in an appropriate amount twice a day for 14 days. 30 days after the completion of the test above, the subjects were crossed over each other, and the same test was carried out at the site different from the previous site to keep the equality of the both of the test groups. The test was controlled under double-blinded.

As a result, six out of ten subjects answered that the skin lotion of the Example had superior moisturization feeling and feelings of the alleviation of wrinkles and sag compared with those containing no phosphorylated saccharide. In addition, six out of ten subjects answered that the skin lotion of the Example had superior feelings of improving skin clearness.

### (Example 23: Emulsion)

According to the following formulation, an emulsion was prepared by a conventional method.

**(Table 4)**

| Emulsion composition | | |
|---|---|---|
| **Component name** | **Function** | **Blending amount (% by weight)** |
| **Stearic acid** | | **3.0** |
| **Cetyl alcohol** | | **2.0** |
| **Vaseline** | | **5.0** |
| **Liquid paraffin** | | **10.0** |
| **Polyoxyethylene (10) monooleate ester** | | **2.0** |
| **Triethanolamine** | **Moisturizing agent** | **1.0** |
| **Sodium panthetheine-S-sulfonate** | **Moisturizing agent** | **10.0** |
| **N,N-Dimethyl PABA octyl ester** | **Ultraviolet absorbing agent** | **5.0** |
| **Hydroquinone monomethyl ether** | **Moisturizing agent (whitening component)** | **0.01** |
| **Sodium bisulfite** | | **1.00** |
| **Azelaic acid** | **Moisturizing agent** | **0.2** |
| **Pyridoxine** | **Cell-activating agent** | **0.2** |
| **Phosphorylated saccharide sodium salt** | | **5.0** |
| **Perfume** | | **Appropriate amount** |
| **Preservative** | | **Appropriate amount** |
| **Ion-exchanged water** | | **Remaining amount** |

Aforementioned emulsion of the present invention was applied to the faces of half of the volunteer subjects (5 men and 5 women, total 10 persons, aged 30 to 46 year old), while a comparative emulsion without the phosphorylated saccharide was applied to the same site of the remaining other half subjects. The 10 subjects applied repeatedly the emulsion in an appropriate amount twice a day for 21 days. 30 days after the completion of the test above, the subjects were crossed over each other, and the same test was carried out at the site different from the previous site to keep the equality of the both of the test groups. The test was controlled under double-blinded.

As a result, seven out of ten subjects answered that the emulsion of the Example had superior moisturization feeling and feelings of the alleviation of wrinkles and sag compared with those containing no phosphorylated saccharide. In addition, eight out of ten subjects answered that the emulsion of the Example had superior feelings of improving skin clearness.

### (Example 24: Emulsion)

According to the following formulation, an emulsion was prepared by a conventional method.

**(Table 5)**

| Emulsion composition | | |
|---|---|---|
| **Component name** | **Function** | **Blending amount (% by weight)** |
| **Jajoba oil ^{*1}** | **Moisturizing agent** | **4.0** |
| **Olive oil ^{*2}** | | **2.0** |
| **Squalene** | | **2.0** |
| **Cetanol** | | **2.0** |
| **Glyceryl monostearate** | | **2.0** |
| **Polyoxyethylene cetyl ether (20E.O)** | | **2.5** |
| **Polyoxyethylene sorbitan oleate (20E.O)** | | **2.0** |
| **1,3-Butylene glycol** | **Moisturizing agent** | **3.0** |
| **L-Ascorbic acid** | **Moisturizing agent (whitening component)** | **1.0** |
| **Reduced phosphorylated saccharide potassium salt** | | **5.0** |
| **Perfume** | | **Appropriate amount** |
| **Preservative** | | **Appropriate amount** |
| **Ion-exchanged water** | | **Remaining amount** |

| | | |
|---|---|---|
| ^{*1} Jajoba oil made in America was used as the jajoba oil. ^{*2} An Olivemanon virgin oil (manufactured by Nippon Olive Co., Ltd.) was used as the olive oil. | | |

Aforementioned emulsion of the present invention was applied to the faces of half of the volunteer subjects (6 men and 4 women, total 10 persons, aged 24 to 39 year old), while a comparative emulsion without the phosphorylated saccharide was applied on the same site of the remaining other half subjects. The 10 subjects applied repeatedly the emulsion in an appropriate amount twice a day for 14 days. 30 days after the completion of the test above, the subjects were crossed over each other, and the same test was carried out at the side different from the previous site to keep the equality of the both of the test groups. The test was controlled under double-blinded.

As a result, eight out of ten subjects answered that the emulsion of the Example had superior moisturization feeling and feelings of the alleviation of wrinkles and sag compared with those containing no phosphorylated saccharide. In addition, six out of ten subjects answered that the emulsion of the Example had superior feelings of improving skin clearness.

### (Example 25: Powder)

According to the following formulation, a powder was prepared by a conventional method.

**(Table 6)**

| Powder composition | | |
|---|---|---|
| **Component name** | **Function** | **Blending amount (% by weight)** |
| **Tranexamic acid** | **Anti-inflammatory component** | **0.1** |
| **Calamine** | | **0.1** |
| **Sulfur** | **Moisturizing agent (anti-inflammatory component)** | **0.1** |
| **Oil-soluble licorice extract ^{*1}** | **Moisturizing agent** | **1.0** |
| **Dextrin** | | **2.0** |
| **Talc** | | **95.0** |
| **Decaglycel stearate** | | **1.0** |
| **Phosphorylated saccharide zinc salt** | | **1.0** |

| | | |
|---|---|---|
| ^{*1} An oil-soluble licorice extract (manufactured by Maruzen Pharmaceuticals Co., Ltd.) was used as the oil-soluble licorice extract. | | |

Aforementioned powder of the present invention was applied to the faces of half of the volunteer subjects (5 men and 5 women, total 10 persons, aged 25 to 35 year old), while a comparative powder without the phosphorylated saccharide was applied to the same site of the remaining other half subjects. The 10 subjects applied repeatedly the powder in an appropriate amount twice a day for 21 days. 30 days after the completion of the test above, the subjects were crossed over each other, and the same test was carried out at the site different from the previous site to keep the equality of the both of the test groups. The test was controlled under double-blinded.

As a result, six out of ten subjects answered that the formulation of the Example had superior moisturization feeling and feelings of the alleviation of wrinkles and sag compared with the same formulation except that it contains no phosphorylated saccharide.

### (Example 26: Facial mask)

According to the following formulation, a facial mask was prepared by a conventional method.

**(Table 7)**

| Jelly facial mask composition | | |
|---|---|---|
| **Component name** | **Function** | **Blending amount (% by weight)** |
| **Carboxyvinyl polymer** | | **2.0** |
| **Isopropanolamine** | | **0.2** |
| **Colloidal kaolin** | | **20.0** |
| **Rosemary extract ^{*1}** | **Moisturizing agent** | **0.5** |
| **Zinc oxide** | **Anti-inflammatory component** | **5.0** |
| **Gelatin** | | **2.0** |
| **Glycerol** | **Moisturizing agent** | **20.0** |
| **Ethanol** | | **15.0** |
| **Phosphorylated saccharide calcium salt** | | **5.0** |
| **Perfume** | | **Appropriate amount** |
| **Preservative** | | **Appropriate amount** |
| **Ion-exchanged water** | | **Remaining amount** |

| | | |
|---|---|---|
| ^{*1} Pharcolex rosemary E (manufactured by Ichimaru Pharcos Co., Ltd.) was us as the rosemary extract. | | |

Aforementioned facial mask of the present invention was applied to the faces of half of the volunteer subjects (6 men and 4 women, total 10 persons, aged 26 to 38 year old), while a comparative facial mask without the phosphorylated saccharide was applied to the same site of the remaining other half subjects. After drying for about 20 minutes, it was washed away. The 10 subjects applied repeatedly the facial mask in an appropriate amount once a day for 14 days. 30 days after the completion of the test above, the subjects were crossed over each other, and the same test was carried out at the site different from the previous site to keep the equality of the both of the test groups. The test was controlled under double-blinded.

As a result, eight out of ten subjects answered that the formulation of the Example had superior moisturization feeling and feelings of the alleviation of wrinkles and sag compared with the same formulation except that it contains no phosphorylated saccharide.

### (Example 27: Facial mask)

According to the following formulation, a facial mask was prepared by a conventional method.

**(Table 8)**

| Jelly facial mask composition | | |
|---|---|---|
| **Component name** | **Function** | **Blending amount (% by weight)** |
| **Carboxyvinyl polymer** | | **2.0** |
| **Isopropanolamine** | | **0.2** |
| **Colloidal kaolin** | | **20.0** |
| **Zinc oxide** | **Anti-inflammatory component** | **5.0** |
| **Gelatin** | | **2.0** |
| **Glycerol** | **Moisturizing agent** | **20.0** |
| **Ethanol** | | **15.0** |
| **Reduced phosphorylated saccharide magnesium salt** | | **1.0** |
| **Perfume** | | **Appropriate amount** |
| **Preservative** | | **Appropriate amount** |
| **Ion-exchanged water** | | **Remaining amount** |

Aforementioned facial mask of the present invention was applied to the faces of half of the volunteer subjects (6 men and 4 women, total 10 persons, aged 26 to 38 year old), while a comparative facial mask without the phosphorylated saccharide was applied to the same site of the remaining other half subjects. After drying for about 20 minutes, it was washed away. The 10 subjects applied repeatedly the facial mask in an appropriate amount once a day for 21 days. 30 days after the completion of the test above, the subjects were crossed over each other, and the same test was carried out at the site different from the previous site to keep the equality of the both of the test groups. The test was controlled under double-blinded.

As a result, six out of ten subjects answered that the facial mask of the Example had superior moisturization feeling and feelings of the alleviation of wrinkles and sag compared with those containing no phosphorylated saccharide.

### (Example 28: Cream)

According to the following formulation, a cream was prepared by a conventional method.

**(Table 9)**

| Cream composition | | |
|---|---|---|
| **Component name** | **Function** | **Blending amount (% by weight)** |
| **Propylene glycol** | **Moisturizing agent** | **5.0** |
| **Beeswax** | | **4.0** |
| **Cetyl alcohol** | | **5.0** |
| **Reduced lanolin** | | **5.0** |
| **Squalane** | | **36.0** |
| **Glyceryl monostearate** | | **2.0** |
| **OPOE (20) sorbitan monolaurate** | | **2.0** |
| **Methylparaben** | | **0.1** |
| **Ethylparaben** | | **0.2** |
| **Allantoin** | **Anti-inflammatory component (whitening component)** | **3.0** |
| **Japanese angelica root extract ^{*1}** | **Moisturizing agent (whitening component)** | **0.2** |
| **Crude sugar extract ^{*2}** | **Moisturizing agent** | **1.0** |
| **Teprenone** | **whitening component** | **1.0** |
| **Reduced phosphorylated saccharide magnesium salt** | | **1.0** |
| **Perfume** | | **0.1** |
| **Preservative** | | **Appropriate amount** |
| **Ion-exchanged water** | | **Remaining amount** |

| | | |
|---|---|---|
| ^{*1} An Japanese angelica root extract BG-J (manufactured by Maruzen Pharmaceuticals, Co., Ltd.) was used as the Japanese angelica root extract. ^{*2} The crude sugar extract used was prepared according to the method described in Japanese Laid-open Patent Publication No. 60-78912. | | |

Aforementioned cream of the present invention was applied to the internal side of the right upper arms of half of the volunteer subjects (5 men and 5 women, total 10 persons, aged 24 to 39 year old), while a comparative cream without the phosphorylated saccharide was applied to the same site of the remaining other half subjects. The 10 subjects applied repeatedly the cream of an appropriate amount twice a day for 30 days. 30 days after the completion of the test above, the subjects were crossed over each other, and the same test was carried out at the site different from the previous site to keep the equality of the both of the test groups. The test was controlled under double-blinded.

As a result, seven out of ten subjects answered that the cream of the Example had superior moisturization feeling and feelings of the alleviation of wrinkles and sag compared with those containing no phosphorylated saccharide. In addition, eight out of ten subjects answered that the cream of the Example had superior feelings of improving skin clearness.

### (Example 29: Hand cream)

According to the following formulation, a hand cream was prepared by a conventional method.

**(Table 10)**

| Hand cream composition | | |
|---|---|---|
| **Component name** | **Function** | **Blending amount (% by weight)** |
| **Stearic acid** | | **8.0** |
| **Cetyl palmitate** | | **2.0** |
| **Cetanol** | | **3.0** |
| **Lanolin** | | **1.0** |
| **Liquid paraffin (light)** | | **15.0** |
| **Silicone oil** | | **1.0** |
| **Polyoxyethylene (20) sorbitan monolaurate** | | **1.0** |
| **Triethanolamine** | **Moisturizing agent** | **1.0** |
| **Propylene glycol** | **Moisturizing agent** | **5.0** |
| **Sorbitol (70%)** | **Moisturizing agent** | **2.0** |
| **Phosphorylated saccharide magnesium salt** | | **1.0** |
| **Perfume** | | **0.1** |
| **Preservative** | | **Appropriate amount** |
| **Ion-exchanged water** | | **Remaining amount** |

Aforementioned hand cream of the present invention was applied to the back of the hands of half of the volunteer subjects (5 men and 5 women, total 10 persons, aged 27 to 42 year old), while a comparative hand cream without the phosphorylated saccharide was applied to the same site of the remaining other half subjects. The 10 subjects applied repeatedly the hand cream in an appropriate amount twice a day for 21 days. 30 days after the completion of the test above, the subjects were crossed over each other, and the same test was carried out at the site different from the previous site to keep the equality of the both of the test groups. The test was controlled under double-blinded.

As a result, seven out of ten subjects answered that the cream of the Example had superior moisturization feeling and feelings of the alleviation of wrinkles and sag compared with those containing no phosphorylated saccharide.

### (Example 30: cosmetic preparation for scalp)

According to the following formulation, a cosmetic preparation for scalp was prepared by a conventional method.

**(Table 11)**

| Cosmetic preparation for scalp (scalp treatment) | | |
|---|---|---|
| **Component name** | **Function** | **Blending amount (% by weight)** |
| **1,3-Butylene glycol** | **Moisturizing agent** | **6.0** |
| **Polyethylene glycol** | | **4.0** |
| **Ethanol** | | **11.0** |
| **POE (60) hydrogenated castor oil** | | **2.0** |
| **Caustic potash** | | **0.1** |
| **Carboxyvinyl polymer** | | **0.2** |
| **Hexyl decyl palmitate** | | **11.0** |
| **Squalane** | | **5.0** |
| **Beeswax** | | **0.5** |
| **Allantoin** | **Anti-inflammatory component (whitening component)** | **4.0** |
| **Preservative** | | **0.2** |
| **Phosphorylated saccharide magnesium salt** | | **1.0** |
| **Perfume** | | **0.1** |
| **Ion-exchanged water** | | **Remaining amount** |

Aforementioned cosmetic preparation for scalp of the present invention was applied to scalps of half of the volunteer subjects (6 men and 4 women, total 10 persons, aged 26 to 38 year old), while a comparative cosmetic preparation for scalp without the phosphorylated saccharide was applied to the same site of the remaining other half subjects. The 10 subjects was applied repeatedly 1 ml of the cosmetic preparation once a day for 30 days. 30 days after the completion of the test above, the subjects were crossed over each other, and the same test was carried out at the site different from the previous site to keep the equality of the both of the test groups. The test was controlled under double-blinded.

As a result, six out of ten subjects answered that the cosmetic preparation of the Example had superior in feelings of the alleviation of moisturization compared with those containing no phosphorylated saccharide.

### (Example 31: Ointment)

According to the following formulation, an ointment was prepared by a conventional method.

**(Table 12)**

| Ointment | | |
|---|---|---|
| **Component name** | **Function** | **Blending amount (% by weight)** |
| **Vaseline** | | **40.0** |
| **Stearyl alcohol** | | **15.0** |
| **Japan tallow** | | **15.0** |
| **POE (10) oleate** | | **0.25** |
| **Glyceryl monostearate** | | **0.25** |
| **Allantoin** | **Anti-inflammatory component (whitening component)** | **1.0** |
| **Sorbitol** | **Moisturizing agent** | **5.0** |
| **Propylene glycol** | | **5.0** |
| **Gentiana extract ^{*1}** | **Moisturizing agent** | **0.3** |
| **Reduced phosphorylated saccharide zinc salt** | | **6.0** |
| **Preservative** | | **Appropriate amount** |
| **Ion-exchanged water** | | **Remaining amount** |

| | | |
|---|---|---|
| ^{*1} Gentiana extract liquid BG (manufactured by Maruzen Pharmaceuticals, Co., Ltd.) was used as the Gentiana extract. | | |

Aforementioned ointment of the present invention was applied to the back of the hands of half of the volunteer subjects (6 men and 4 women, total 10 persons, aged 26 to 38 year old), while a comparative ointment without the phosphorylated saccharide was applied to the same site of the remaining other half subjects. The 10 subjects applied repeatedly the ointment in an appropriate amount twice a day for 14 days. 30 days after the completion of the test above, the subjects were crossed over each other, and the same test was carried out at the site different from the previous site to keep the equality of the both of the test groups. The test was controlled under double-blinded.

As a result of repeated use of both ointments, eight out of ten subjects answered that the ointment of the Example had superior moisturization feeling and feelings of the alleviation of wrinkles and sag compared with those containing no phosphorylated saccharide. In addition, six out of ten subjects answered that the ointment of the Example had superior feelings of improving skin clearness.

The external preparation for skins obtained in Examples 20 to 31 were all excellent in moisturizing effect, lower in skin irritation and sensitization, and excellent in stability over time.

### (Test Example 1)

A clinical test was carried out by using a cream according to the present invention in the same formulation as that of Example 9, except that the amount of the reduced phosphorylated saccharide magnesium salt was changed from 1.0 % by weight to 0.5 % by weight, and a comparative cream in the same composition as that of Example 9 except that the reduced phosphorylated saccharide magnesium salt was replaced with oligotose.

Aforementioned cream of the present invention was applied on the internal side of the right upper arms of half (3 men and 3 women, total 6 persons) of 12 volunteer subjects (6 men and 6 women, total 12 persons, aged 25 to 55 year old), while the comparative cream was applied to the same site of the remaining other half subjects. It was applied three times a day (every 8 hours) for 7 days consecutively, and the water vaporization amount and the water content were measured. 30 days after the completion of the clinical test above, the subjects were crossed over each other, and the same test was carried out at the site different from the previous site of internal side of the right upper arms to keep the equality of the both of the test groups. The test was controlled under double-blind. The results are shown in Table 14.

**(Table 14)**

| 6 man and 6 women, total 12 persons, aged 25 to 55 year old | | |
|---|---|---|
| | **Comparative cream** | **Cream of the present invention** |
| **Very effective** | **1 subject (8.3%)** | **3 subjects (25.0%)** |
| **Effective** | **3 subjects (25.0%)** | **5 subjects (41.7%)** |
| **Slightly effective** | **6 subjects (50.0%)** | **3 subjects (25.0%)** |
| **Not effective** | **2 subjects (16.7%)** | **1 subject (8.3%)** |
| **Worsened** | **none** | **none** |

The cream according to the present invention is superior in skin-moisturizing effect compared to the cream containing oligotose. No adverse effect was observed. Thus, it is understood that the cream according to the present invention is excellent.

### (Example 33: influence of combined use of phosphorylated saccharide calcium salt and ascorbic acid on collagen induction by human skin fibroblast)

Normal human fibroblast cells were adjusted with 10% FBS-DMEM (manufactured by Invitrogen) to a density of 2.5×10⁴ cells per well, and then preincubated on the 96-well plate for 24 hours. After removal of the medium, 4% FBS-DMEM (control), 4% FBS-DMEM containing 1% phosphorylated saccharide calcium salt, 4% FBS-DMEM containing 0.0044% ascorbic acid, or 4% FBS-D-MEM containing 1% phosphorylated saccharide calcium salt and 0.0044% ascorbic acid was added to each well, and cultured at 37°C under 5% CO₂ for 72 hours. After the completion of the culture, the amount of collagen in the medium was measured. The amount of collagen was measured by using a Sircol collagen assay Kit (manufactured by Biocolor Ltd.) . The amount of collagen in the medium of the well containing 1% phosphorylated saccharide calcium salt was 1.2 µg larger than that obtained from the control. The amount of collagen in the medium of the well containing 0.0044% ascorbic acid was increased by 1.4 µg, compared to that of the control well. On the other hand, the amount of collagen in the medium of the well containing both 1% phosphorylated saccharide calcium salt and ascorbic acid was increased by 4.7 µg, compared to that of the control well. It was confirmed that combined use of the phosphorylated saccharide calcium salt and ascorbic acid attained the increase of collagen increasement by about 1.8 times, compared to the total of increased collagen amount when the phosphorylated saccharide calcium salt or ascorbic acid was used alone. In other words, a synergic effect of inducing collagen using the phosphorylated saccharide calcium salt in combination with ascorbic acid was confirmed.

### INDUSTRIAL APPLICABILITY

An external preparation for skin which enhances moisturizing effect is realized by blending a mineral salt of an phosphorylated saccharide in an external preparation for skin, for example, a cosmetic product and quasi-drug, such as emulsion, skin lotion, cream, shampoo and face wash. Furthermore, this external preparation for skin is also possible to supply a mineral needed for the skin effectively and stably. Accordingly, it is possible to develop an effective external preparation for skin that can improve skin aging, give practically effective wrinkle formation treatment method, can be used safely to the skin, and supply needed minerals in a state stably dissolved in the external preparation for skin.

## Claims

1. Use of an external preparation for improving skin firmness, wrinkles or elasticity; alleviating dry skin; delaying aging phenomena; delaying dullness or sag; moisturizing skin; or whitening skin by removing melanin and active oxygen generated by UV irradiation, said external preparation comprising a inorganic salt of a phosphorylated saccharide, wherein the saccharide moiety in the phosphorylated saccharide is a glucan or reduced glucan having the degree of polymerization of 2 or more and 100 or less.

2. Use according to Claim 1, wherein the inorganic salt is a calcium, magnesium, potassium, zinc, iron or sodium salt.

3. Use according to Claim 1 or 2, wherein the external preparation further comprises a moisturizing agent.

4. Use according to Claim 3, wherein the moisturizing agent is an ascorbic acid or an ascorbic acid derivative.

5. Use according to any of Claims 1-4, wherein the external preparation is selected from the group consisting of skin lotion, emulsion, cream, emollient cream, cold cream, vanishing cream, facial mask, gel, face pack, foundation, cake, rouge for cheek, eye shadow, lipstick, lip cream, gloss, soap, body soap, face wash, shampoo, rinse, conditioner, hair treatment, hair lotion and shaving cream.

6. Use according to Claim 1, wherein the external preparation comprises a second component which is selected from the group consisting of moisturizing agents, whitening components, and antioxidants.

7. Use according to Claim 6, wherein the second component is a moisturizing agent, and the moisturizing agent is selected from the group consisting of ascorbic acid and the derivatives thereof, vitamins other than ascorbic acid, pyridoxine derivatives, α-tocopherol derivatives, pan-tothenic acid derivatives, saccharides and saccharide derivatives, amino acids and the derivatives thereof, polyhydric alcohols, phenol and the derivatives thereof, collagens, hydroxycarboxylic acids and the salts thereof, hydroxysalicylic acid glycosides, hydroxysalicylic acid aliphatic ester glycosides, hydroxycinnamic acid and the derivatives thereof, caffeic acid and the derivatives thereof, crude drug extracts, natural extracts, placenta extract, oil-soluble licorice extract, ceramides, ceramide analogues, crude sugar extracts, molasses extracts, mycelia culture and the extracts thereof, urea, hinokitiol, sulfur, Azelain and the derivatives thereof, vitamin E-nicotinate and diisopropylamine dichloroacetate.

8. Use according to Claim 6, wherein the second component is a whitening component, and the whitening component is selected from the group consisting of tyrosinase inhibitor, endothelin antagonist, α-MSH inhibitor, α-arbutin, arbutins, the salts and derivatives thereof, ascorbic acid and the derivatives thereof, ellagic acid-based compounds and the alkali-metal salts thereof, kojic acid and the derivatives thereof, resorcinol derivatives, nordihy-droguaiaretic acid, teprenone, allantoin, aminoethyl compounds, alkylenediamine carboxylic acid derivatives, betaine derivatives, acylmethyltaurines, hederacoside, gymnema saponins, beet saponins, γ-pyrrone glycosides, biphenyl compounds, sodium bisulfite, fibronectins, and plant extracts.

9. Use according to Claim 6, wherein the second component is an antioxidant, and the antioxidant is selected from the group consisting of vitamin A's, the derivatives and salts thereof; carotenoids and the derivatives thereof; vitamin B's, the derivatives and salts thereof; vitamin C's, the derivatives and salts thereof; vitamin D's, the derivatives and salts thereof; vitamin E's, the derivatives and salts thereof; trolox, the derivatives and the salts thereof; dihydroxytoluene, butylhydroxytoluene, butylhydroxyanisole, dibutyl-hydroxytoluene, α-lipoic acid, dehydrolipoic acid, glutathione, the derivatives and salts thereof; uric acid; erythorbic acid, the derivatives and salts thereof; gallic acid, the derivatives and salts thereof; rutin, the derivatives and salts thereof; tryptophan, the derivatives and salts thereof; histidine, the derivatives and salts thereof; cysteine derivatives and salts thereof; cystine derivatives and the salts thereof; carnosine, the derivatives and salts thereof; homocarnosine, the derivatives and salts thereof; anserine, the derivatives and salts thereof; carcinin, the derivatives and salts thereof; dipeptide or tripeptide derivatives containing histidine and/or tryptophan and/or histamine and the salts thereof; flavonoids; tannic acid; caffeic acid; ferulic acid; protocatechuic acid; chalcone; oryzanol; carnosol; sesamol; sesamine; sesamolin; gingerone; curcumin; tetrahydrocurcumin; clovamide; deoxyclovamide; shogaols; capsaicin; vanillylamide; ellagic acid; bromphenol; flavoglassine; melanoidin; riboflavin; riboflavin butyrate esters; flavin mononucleotide; flavin adenine nucleotide; ubiquinone; ubiquinol; mannitol; bilirubin; cholesterol; ebselene; seleno-methionine; ceruloplasmin; transferrin; lactoferrin; albumin; bilirubin; superoxide dismutase; catalase; glutathione peroxidase; metallothionein; O-phosphono-pyridoxylidene rhodamine; N-(2-hydroxybenzyl)amino acids, the derivatives and salts thereof; and N-(4-pyridoxylmethylene)amino acids, the derivatives and salts thereof.

10. Use according to Claim 6, wherein the phosphorylated saccharide is an inorganic salt of a phosphorylated saccharide.

11. Use according to Claim 6, wherein the phosphorylated saccharide is a calcium, magnesium, potassium, zinc, iron or sodium salt.

12. Use according to Claim 6, wherein the external preparation is selected from the group consisting of skin lotion, emulsion, cream, emollient cream, cold cream, vanishing cream, facial mask, gel, face pack, foundation, cake, rouge for cheek, eye shadow, lipstick, lip cream, gloss, soap, body soap, face wash, shampoo, rinse, conditioner, hair treatment, hair lotion and shaving cream.

13. An external preparation for use in a method of increasing skin moisturization and thus activating skin metabolism, wherein the external preparation comprises: an inorganic salt of a phosphorylated saccharide, wherein the saccharide moiety in the phosphorylated saccharide is a glucan or reduced glucan having a degree of polymerization of 2 or more and 100 or less.

14. An external preparation for use in a method of claim 13, wherein the external preparation comprises a second component, wherein the second component is selected from the group consisting of moisturizing components, whitening components, ultraviolet absorbents, anti-inflammatory agents, cell-activating agents and antioxidants.

15. An external preparation for use as a medicament comprising: an inorganic salt of a phosphorylated saccharide, wherein the saccharide moiety in the phosphorylated saccharide is a glucan or reduced glucan having the degree of polymerization of 2 or more and 100 or less.

16. An external preparation for use according to claim 15, wherein the external preparation comprises a second component which is selected from the group consisting of moisturizing components, whitening components, ultraviolet absorbents, anti-inflammatory agents, cell-activating agents and antioxidants.

## Patentansprüche

1. Verwendung einer externen Zubereitung zur Verbesserung von Hautfestigkeit, Falten oder Elastizität; zur Linderung von trockener Haut; zur Verzögerung von Alterungserscheinungen; zur Verzögerung von Mattheit oder Erschlaffung; zur Befeuchtung der Haut; oder zur Aufhellung der Haut durch Entfernen von Melanin und aktivem Sauerstoff, welcher durch UV-Bestrahlung erzeugt wird; wobei die externe Zubereitung ein anorganisches Salz eines phosphorylierten Saccharids umfasst, und wobei der Saccharidrest in dem phosphorylierten Saccharid ein Glucan oder ein reduziertes Glucan mit einem Polymerisationsgrad von 2 oder mehr und 100 oder weniger ist.

2. Verwendung nach Anspruch 1, wobei das anorganische Salz ein Calcium-, Magnesium-, Kalium-, Zink-, Eisen- oder Natriumsalz ist.

3. Verwendung nach Anspruch 1 oder 2, wobei die externe Zubereitung ferner ein Feuchtigkeitsmittel umfasst.

4. Verwendung nach Anspruch 3, wobei das Feuchtigkeitsmittel eine Ascorbinsäure oder ein Ascorbinsäure-Derivat ist.

5. Verwendung nach einem der Ansprüche 1-4, wobei die externe Zubereitung ausgewählt ist aus der Gruppe bestehend aus Hautlotion, Emulsion, Creme, Weichmachercreme, Cold Cream, Tagescreme, Gesichtsmaske, Gel, Gesichtspackung, Grundierung, Cake Make-up, Wangenrouge, Lidschatten, Lippenstift, Lippencreme, Gloss, Seife, Körperseife, Gesichtsreinigung, Shampoo, Spülung, Haarkur, Haarpflege, Haarlotion und Rasiercreme.

6. Verwendung nach Anspruch 1, wobei die externe Zubereitung eine zweite Komponente umfasst, die ausgewählt ist aus der Gruppe bestehend aus feuchtigkeitsspendenden Mitteln, aufhellenden Komponenten und Antioxidantien.

7. Verwendung nach Anspruch 6, wobei die zweite Komponente ein Feuchtigkeitsmittel ist und das Feuchtigkeitsmittel ausgewählt ist aus der Gruppe bestehend aus Ascorbinsäure und Derivaten davon, Vitaminen, bei denen es sich nicht um Ascorbinsäure handelt, Pyridoxin-Derivaten, α-Tocopherol-Derivaten, Pantothensäure-Derivaten, Sacchariden und Saccharid-Derivaten, Aminosäuren und Derivaten davon, mehrwertigen Alkoholen, Phenol und Derivaten davon, Kollagenen, Hydroxycarbonsäuren und Salzen davon, Hydroxysalicylsäureglycosiden, aliphatischen Esterglycosiden von Hydroxysalicylsäure, Hydroxyzimtsäure und Derivaten davon, Kaffeesäure und Derivaten davon, Wirkstoff-Rohextrakten, natürlichen Extrakten, Plazentaextrakten, öl-löslichem Süßholzextrakt, Ceramiden, Ceramid-Analoga, Rohzucker-Extrakten, Melasse-Extrakten, Myzelienkultur und Extrakten davon, Harnstoff, Hinokitiol, Schwefel, Azelain und Derivaten davon, Vitamin E-Nicotinat und Diisopropylamindichloracetat.

8. Verwendung nach Anspruch 6, wobei die zweite Komponente eine aufhellende Komponente ist, und die aufhellende Komponente ausgewählt ist aus der Gruppe bestehend aus Tyrosinase-Inhibitor, Endothelin-Antagonist, α-MSH-Inhibitor, α-Arbutin, Arbutinen, Salzen und Derivaten davon, Ascorbinsäure und Derivaten davon, auf Ellagsäure basierenden Verbindungen und den Alkalimetallsalzen davon, Kojisäure und Derivaten davon, Resorcin-Derivaten, Nordihydroguajaretsäure, Teprenon, Allantoin, Aminoethyl-Verbindungen, Alkylendiamincarbonsäure-Derivaten, Betain-Derivaten, Acylmethyltaurinen, Hederacosiden, Gymnema-Saponinen, Rübensaponinen, γ-Pyron-Glycoside, Biphenyl-Verbindungen, Natriumbisulfit, Fibronektinen und Pflanzenextrakten.

9. Verwendung nach Anspruch 6, wobei die zweite Komponente ein Antioxidans ist und das Antioxidans ausgewählt ist aus der Gruppe bestehend aus A-Vitaminen und Derivaten und Salzen davon; Carotinoiden und Derivaten davon; B-Vitaminen und Derivaten und Salzen davon; C-Vitaminen und Derivaten und Salzen davon; D-Vitaminen und Derivaten und Salzen davon; E-Vitaminen und Derivaten und Salzen davon; Trolox und Derivaten und Salzen davon, Dihydroxytoluol, Butylhydroxytoluol, Butylhydroxyanisol, Dibutylhydroxytoluol, α-Liponsäure, Dihydroliponsäure, Glutathion und den Derivaten und Salzen davon; Harnsäure; Erythorbinsäure und den Derivaten und Salzen davon; Gallussäure und den Derivaten und Salzen davon; Rutin und den Derivaten und Salzen davon; Tryptophan und den Derivaten und Salzen davon; Histidin und den Derivaten und Salzen davon; Cystein-Derivaten und Salzen davon; Cystin-Derivaten und Salzen davon; Carnosin und den Derivaten und Salzen davon; Homocarnosin und den Derivaten und Salzen davon; Anserin und den Derivaten und Salzen davon; Carcinin und den Derivaten und Salzen davon; Dipeptid- oder Tripeptid-Derivaten, welche Histidin und/oder Tryptophan und/oder Histamin enthalten und die Salze davon; Flavonoiden; Gerbsäure; Kaffeesäure; Ferulasäure; Protocatechusäure; Chalcon; Oryzanol; Carnosol; Sesamol; Sesamin; Sesamolin; Gingeron; Curcumin; Tetrahydrocurcumin; Clovamid; Desoxyclovamid; Shogaolen; Capsaicin; Vanillylamid; Ellagsäure; Bromphenol; Flavoglassin; Melanoidin; Riboflavin; Riboflavinbutyratester; Flavin-Mononukleotid; Flavin-Adenin-Nucleotid; Ubichinon; Ubiquinol; Mannitol; Bilirubin; Cholesterin; Ebselen; Selenomethionin; Ceruloplasmin; Transferrin; Lactoferrin; Albumin; Bilirubin; Superoxid-Dismutase; Katalase; Glutathion-Peroxidase; Metallothionein; O-Phosphono-Pyridoxyliden-Rhodamin; N-(2-Hydroxybenzyl)-Aminosäuren und Derivaten und Salzen davon; und N-(4-Pyridoxylmethylen)-Aminosäuren und Derivaten und Salzen davon.

10. Verwendung nach Anspruch 6, wobei das phosphorylierte Saccharid ein anorganisches Salz eines phosphorylierten Saccharids ist.

11. Verwendung nach Anspruch 6, wobei das phosphorylierte Saccharid ein Calcium-, Magnesium-, Kalium-, Zink-, Eisen- oder Natriumsalz ist.

12. Verwendung nach Anspruch 6, wobei die externe Zubereitung ausgewählt ist aus der Gruppe bestehend aus Hautlotion, Emulsion, Creme, Weichmachercreme, Cold Cream, Tagescreme, Gesichtsmaske, Gel, Gesichtspackung, Grundierung, Cake Make-up, Wangenrouge, Lidschatten, Lippenstift, Lippencreme, Gloss, Seife, Körperseife, Gesichtsreinigung, Shampoo, Spülung, Haarkur, Haarpflege, Haarlotion und Rasiercreme.

13. Externe Zubereitung zur Verwendung in einem Verfahren zur Erhöhung der Hautfeuchtigkeit und somit zur Aktivierung des Hautmetabolismus, wobei die externe Zubereitung ein anorganisches Salz eines phosphorylierten Saccharids umfasst, und wobei der Saccharidrest in dem phosphorylierten Saccharid ein Glucan oder ein reduziertes Glucan mit einem Polymerisationsgrad von 2 oder mehr und 100 oder weniger ist.

14. Externe Zubereitung zur Verwendung in einem Verfahren nach Anspruch 13, wobei die externe Zubereitung eine zweite Komponente umfasst, und wobei die zweite Komponente ausgewählt aus der Gruppe bestehend aus feuchtigkeitsspendenden Komponenten, aufhellenden Komponenten, UV-Absorptionsmitteln, antientzündlichen Mitteln, zellaktivierenden Mitteln und Antioxidantien.

15. Externe Zubereitung zur Verwendung als Medikament, die ein anorganisches Salz eines phosphorylierten Saccharids umfasst, wobei der Saccharidrest in dem phosphorylierten Saccharid ein Glucan oder ein reduziertes Glucan mit einem Polymerisationsgrad von 2 oder mehr und 100 oder weniger ist.

16. Externe Zubereitung zur Verwendung nach Anspruch 15, wobei die externe Zubereitung eine zweite Komponente umfasst, die ausgewählt ist aus der Gruppe bestehend aus feuchtigkeitsspendenden Komponenten, aufhellenden Komponenten, UV-Absorptionsmitteln, antientzündlichen Mitteln, zellaktivierenden Mitteln und Antioxidantien.

## Revendications

1. Utilisation d'une préparation à usage externe pour améliorer la fermeté, atténuer les rides ou améliorer l'élasticité de la peau ; réduire la sécheresse de la peau ; retarder le phénomène de vieillissement ; retarder la perte d'éclat ou l'affaissement ; hydrater la peau ; ou éclaircir la peau par élimination de la mélanine et de l'oxygène actif engendré par irradiation UV, ladite préparation à usage externe comprenant un sel inorganique d'un saccharide phosphorylé, le fragment saccharidique dans le saccharide phosphorylé étant un glucane ou glucane réduit ayant un degré de polymérisation de 2 ou plus et de 100 ou moins.

2. Utilisation selon la revendication 1, dans laquelle le sel inorganique est un sel de calcium, magnésium, potassium, zinc, fer ou sodium.

3. Utilisation selon la revendication 1 ou 2, dans laquelle la préparation à usage externe comprend encore un agent hydratant.

4. Utilisation selon la revendication 3, dans laquelle l'agent hydratant est l'acide ascorbique ou un dérivé d'acide ascorbique.

5. Utilisation selon l'une quelconque des revendications 1 à 4, dans laquelle la préparation à usage externe est choisie dans l'ensemble constitué par une lotion pour la peau, une émulsion, une crème, une crème émolliente, une crème cold-cream, une crème de jour, un masque facial, un gel, un masque de beauté, un fond de teint, une poudre compacte, un rouge à joues, un fard à paupières, un rouge à lèvres, une crème pour les lèvres, un lustrant, un savon, un savon pour le corps, un nettoyant pour le visage, un shampooing, un produit de rinçage, un conditionneur, un produit de traitement capillaire, une lotion capillaire et une crème à raser.

6. Utilisation selon la revendication 1, dans laquelle la préparation à usage externe comprend un second composant qui est choisi dans l'ensemble constitué par les agents hydratants, les composants éclaircissants et les antioxydants.

7. Utilisation selon la revendication 6, dans laquelle le second composant est un agent hydratant, et l'agent hydratant est choisi dans l'ensemble constitué par l'acide ascorbique et ses dérivés, les vitamines autres que l'acide ascorbique, les dérivés de pyridoxine, les dérivés d'a-tocophérol, les dérivés d'acide pantothénique, les saccharides et dérivés de saccharides, les acides aminés et leurs dérivés, les alcools polyhydriques, le phénol et ses dérivés, les collagènes, les acides hydroxycarboxyliques et leurs sels, les glycosides à base d'acide hydroxysalicylique, les glycosides à base d'esters aliphatiques d'acide hydroxysalicylique, l'acide hydroxycinnamique et ses dérivés, l'acide caféique et ses dérivés, des extraits médicamenteux bruts, des extraits naturels, l'extrait de placenta, l'extrait de réglisse liposoluble, les céramides, les analogues de céramides, les extraits de sucre bruts, les extraits de mélasse, une culture de mycélium et des extraits d'une telle culture, l'urée, l'hinokitiol, le soufre, l'azélaïne et ses dérivés, le nicotinate de vitamine E et le dichloracétate de diisopropylamine.

8. Utilisation selon la revendication 6, dans laquelle le second composant est un composant éclaircissant, et le composant éclaircissant est choisi dans l'ensemble constitué par un inhibiteur de tyrosinase, un antagoniste d'endothéline, un inhibiteur d'α-MSH, l'a-arbutine, les arbutines, leurs dérivés et sels, l'acide ascorbique et ses dérivés, des composés à base d'acide ellagique et leurs sels de métaux alcalins, l'acide kojique et ses dérivés, les dérivés de résorcinol, l'acide nordihydroguaiarétique, la téprénone, l'allantoïne, les composés aminoéthyle, les dérivés d'acides carboxyliques et alkylènediamines, les dérivés de bétaïne, les acylméthyltaurines, l'hédéracoside, les saponines de gymnéma, les saponines de betterave, les γ-pyrone glycosides, les composés biphényliques, le bisulfite de sodium, les fibronectines et des extraits végétaux.

9. Utilisation selon la revendication 6, dans laquelle le second composant est un antioxydant, et l'antioxydant est choisi dans l'ensemble constitué par les vitamines A, leurs dérivés et sels ; les caroténoïdes et leurs dérivés ; les vitamines B, leurs dérivés et sels ; les vitamines C, leurs dérivés et sels ; les vitamines D, leurs dérivés et sels ; les vitamines E, leurs dérivés et sels ; le trolox, ses dérivés et sels ; le dihydroxytoluène, le butylhydroxytoluène, le butylhydroxyanisole, le dibutyl-hydroxytoluène, l'acide α-lipoïque, l'acide déhydrolipoïque, le glutathion, leurs dérivés et sels ; l'acide urique ; l'acide érythorbique, ses dérivés et sels ; l'acide gallique, ses dérivés et sels ; la rutine, ses dérivés et sels ; le tryptophane, ses dérivés et sels ; l'histidine, ses dérivés et sels ; les dérivés et sels de la cystéine ; les dérivés et sels de la cystine ; la carnosine, ses dérivés et sels ; l'homocarnosine, ses dérivés et sels ; l'ansérine, ses dérivés et sels ; la carcinine, ses dérivés et sels ; des dérivés de dipeptides ou tripeptides contenant de l'histidine et/ou du tryptophane et/ou de l'histamine et leurs sels ; les flavonoïdes ; l'acide tannique ; l'acide caféique; l'acide férulique ; l'acide protocatéchuique ; la chalcone ; l'oryzanol ; le carnosol ; le sésamol ; la sésamine ; la sésamoline ; la gingérone ; la curcumine ; la tétrahydrocurcumine ; le clovamide ; le déoxyclovamide ; les shogaols ; la capsaïcine ; le vanillylamide ; l'acide ellagique ; le bromophénol ; la flavoglassine ; la mélanoïdine ; la riboflavine ; les esters butyriques de riboflavine ; le flavine mononucléotide ; le flavine adénine nucléotide ; l'ubiquinone ; l'ubiquinol ; le mannitol ; la bilirubine ; le cholestérol ; l'ebsélène ; la sélénométhionine ; la céruloplasmine ; la transferrine ; la lactoferrine ; l'albumine ; la bilirubine ; la superoxyde dismutase ; la catalase ; la glutathion peroxydase ; la métallothionéine ; l'O-phosphono-pyridoxylidène rhodamine ; les acides N-(2-hydroxybenzyl)aminés, leurs dérivés et sels ; et les acides N-(4-pyridoxylméthylène)aminés ; leurs dérivés et sels.

10. Utilisation selon la revendication 6, dans laquelle le saccharide phosphorylé est un sel inorganique d'un saccharide phosphorylé.

11. Utilisation selon la revendication 6, dans laquelle le saccharide phosphorylé est un sel de calcium, magnésium, potassium, zinc, fer ou sodium.

12. Utilisation selon la revendication 6, dans laquelle la préparation à usage externe est choisie dans l'ensemble constitué par une lotion pour la peau, une émulsion, une crème, une crème émolliente, une crème cold-cream, une crème de jour, un masque facial, un gel, un masque de beauté, un fond de teint, une poudre compacte, un rouge à joues, un fard à paupières, un rouge à lèvres, une crème pour les lèvres, un lustrant, un savon, un savon pour le corps, un nettoyant pour le visage, un shampooing, un produit de rinçage, un conditionneur, un produit de traitement capillaire, une lotion capillaire et une crème à raser.

13. Préparation à usage externe destinée à l'utilisation dans un procédé d'augmentation de l'hydratation de la peau et ainsi d'activation du métabolisme cutané, où la préparation à usage externe comprend : un sel inorganique d'un saccharide phosphorylé, le fragment saccharidique dans le saccharide phosphorylé étant un glucane ou un glucane réduit ayant un degré de polymérisation de 2 ou plus et 100 ou moins.

14. Préparation à usage externe destinée à l'utilisation dans un procédé selon la revendication 13, où la préparation à usage externe comprend un second composant, le second composant étant choisi dans l'ensemble constitué par les composants hydratants, les composants éclaircissants, les absorbeurs UV, les agents anti-inflammatoires, les agents activant les cellules et les antioxydants.

15. Préparation à usage externe destinée à l'utilisation en tant que médicament, comprenant : un sel inorganique d'un saccharide phosphorylé, le fragment saccharidique dans le saccharide phosphorylé étant un glucane ou un glucane réduit ayant un degré de polymérisation de 2 ou plus et 100 ou moins.

16. Préparation à usage externe destinée à l'utilisation selon la revendication 15, où la préparation externe comprend un second composant qui est choisi dans l'ensemble constitué par les composants hydratants, les composants éclaircissants, les absorbeurs UV, les agents anti-inflammatoires, les agents activant les cellules et les antioxydants.
